(19) Europäisches Patentamt
European Patent Office
Office européen des brevets

(11) Veröffentlichungsnummer: **0 415 886 A2**

# (12) EUROPÄISCHE PATENTANMELDUNG

(21) Anmeldenummer: **90810635.4**

(22) Anmeldetag: **22.08.90**

(51) Int. Cl.5: **C07D 471/04**, C07D 487/04, C07D 473/00, A61K 31/435, A61K 31/495, //(C07D471/04, 235:00,221:00),(C07D487/04, 237:00,221:00),(C07D487/04, 241:00,221:00)

(30) Priorität: **30.08.89 CH 3142/89**

(43) Veröffentlichungstag der Anmeldung:
**06.03.91 Patentblatt 91/10**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Herold, Peter, Dr.**
**Mattweg 19**
**CH-4144 Arlesheim(CH)**
Erfinder: **Bühlmayer, Peter, Dr.**
**Hangstrasse 18**
**CH-4144 Arlesheim(CH)**

## (54) Aza-Verbindungen.

(57) Azabenzimidazol-Verbindungen der Formel

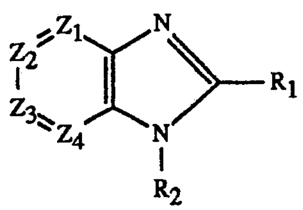

(I),

worin eine oder zwei der Variablen $Z_1$, $Z_2$, $Z_3$ und $Z_4$ für N und die anderen für C(R) stehen, wobei R Halogen, Acyl, gegebenenfalls verestertes oder amidiertes Carboxy oder 5-Tetrazolyl bedeutet oder R für -Z-R′ steht, worin Z für eine Bindung oder für O, $S(O)_m$ oder NH steht, R′ Wasserstoff oder einen aliphatischen Kohlenwasserstoffrest, der gegebenenfalls durch Halogen, Hydroxy, gegebenenfalls substituiertes Amino oder gegebenenfalls verestertes oder amidiertes Carboxy substituiert ist und der gegebenenfalls durch O oder $S(O)_m$ unterbrochen ist, bedeutet und der Index m jeweils für 0, 1 oder 2 steht, $R_1$ einen gegebenenfalls durch Halogen oder Hydroxy substituierten aliphatischen Kohlenwasserstoffrest oder einen cycloaliphatischen oder araliphatischen Kohlenwasserstoffrest bedeutet und $R_2$ für die Gruppe der Formel

EP 0 415 886 A2

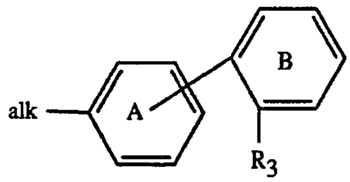

(Ia)

steht, in der alk einen zweiwertigen aliphatischen Kohlenwasserstoffrest bedeutet, $R_3$ COOH, $SO_3H$, Halogenalkansulfonylamino, $PO_2H_2$, $PO_3H_2$ oder 5-Tetrazolyl bedeutet und entweder die Ringe A und B unabhängig voneinander gegebenenfalls durch Halogen, einen gegebenenfalls durch Hydroxy oder Halogen substituierten, gegebenenfalls durch O unterbrochenen, aliphatischen Kohlenwasserstoffrest, gegebenenfalls durch einen aliphatischen Alkohol verethertes Hydroxy oder gegebenenfalls verestertes oder amidiertes Carboxy substituiert sind oder der Ring A durch 5-Tetrazolyl substituiert ist und der Ring B gegebenenfalls wie unmittelbar vorstehend angegeben substituiert ist, in freier Form oder in Salzform, sind in an sich bekannter Weise herstellbar und können beispielsweise als Arzneimittelwirkstoffe verwendet werden.

## AZA-VERBINDUNGEN

Die Erfindung betrifft Azabenzimidazol-Verbindungen der Formel

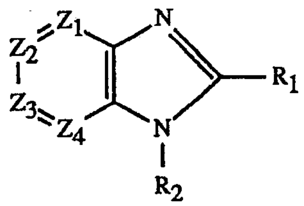

(I),

worin eine oder zwei der Variablen $Z_1$, $Z_2$, $Z_3$ und $Z_4$ für H und die anderen für C(R) stehen, wobei R Halogen, Acyl, gegebenenfalls verestertes oder amidiertes Carboxy oder 5-Tetrazolyl bedeutet oder R für $-Z-R'$ steht, worin Z für eine Bindung oder für $O,S(O)_m$ oder NH steht, $R'$ Wasserstoff oder einen aliphatischen Kohlenwasserstoffrest, der gegebenenfalls durch Halogen, Hydroxy, gegebenenfalls substituiertes Amino oder gegebenenfalls verestertes oder amidiertes Carboxy substituiert ist und der gegebenenfalls durch O oder $S(O)_m$ unterbrochen ist, bedeutet und der Index m jeweils für 0, 1 oder 2 steht, $R_1$ einen gegebenenfalls durch Halogen oder Hydroxy substituierten aliphatischen Kohlenwasserstoffrest oder einen cycloaliphatischen oder araliphatischen Kohlenwasserstoffrest bedeutet und $R_2$ für die Gruppe der Formel

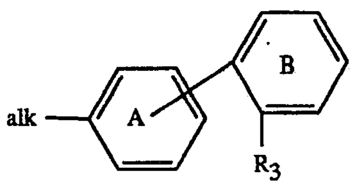

(Ia)

steht, in der alk einen zweiwertigen aliphatischen Kohlenwasserstoffrest bedeutet, $R_3$ COOH, $SO_3H$, Halogenalkansulfonylamino, $PO_2H_2$, $PO_3H_2$ oder 5-Tetrazolyl bedeutet und entweder die Ringe A und B unabhängig voneinander gegebenenfalls durch Halogen, einen gegebenenfalls durch Hydroxy oder Halogen substituierten, gegebenenfalls durch O unterbrochenen, aliphatischen Kohlenwasserstoffrest, gegebenenfalls durch einen aliphatischen Alkohol verethertes Hydroxy oder gegebenenfalls verestertes oder amidiertes Carboxy substituiert sind oder der Ring A durch 5-Tetrazolyl substituiert ist und der Ring B gegebenenfalls wie unmittelbar vorstehend angegeben substituiert ist, in freier Form oder in Salzform, ein Verfahren zur Herstellung dieser Verbindungen, die Verwendung dieser Verbindungen und pharmazeutische Präparate, enthaltend eine solche Verbindung I in freier Form oder in Form eines pharmazeutisch verwendbaren Salzes.

Die Verbindungen der Formel I können als, insbesondere pharmazeutisch verwendbare, Salze vorliegen. Weisen die Verbindungen I mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie gegebenenfalls, z.B. durch Halogen, substituierten $C_1$-$C_4$-Alkan- oder Arylsulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Ferner können die Verbindungen I mit mindestens einer aciden Gruppe (beispielsweise COOH oder 5-Tetrazolyl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono, Di- oder Triniederalkylamin, z. B. Ethyl-, tert.-Butyl-, Diethyl-, Diisopropyl-, Triethyl-, Tributyl- oder Dimethyl-propyl-amin, oder einem Mono-, Di- oder Trihydroxyniederalkylamin, z.B. Mono-, Di- oder Triethanolamin. Weiterhin können entsprechende

innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden werden.

Acyl bedeutet insbesondere Niederalkanoyl.

Verestertes Carboxy bedeutet beispielsweise Carboxy, welches durch einen aliphatischen Alkohol verestert ist, der sich von einem aliphatischen Kohlenwasserstoffrest ableitet, wie von Niederalkyl, Niederalkenyl oder in zweiter Linie Niederalkinyl, welcher gegebenen falls durch O unterbrochen ist, wie von Niederalkoxy-niederalkyl, -niederalkenyl oder -niederalkinyl. Beispielhaft seien Niederalkoxy-, Niederalkenyloxy- und Niederalkoxyniederalkoxy-carbonyl genannt.

Amidiertes Carboxy ist beispielsweise Carbamoyl, in welchem die Aminogruppe gegebenenfalls durch einen aliphatischen oder araliphatischen Kohlenwasserstoffrest, wie Niederalkyl, Niederalkenyl, Niederalkinyl oder Phenyl-niederalkyl, -niederalkenyl oder -niederalkinyl, unabhängig voneinander mono- oder disubstituiert oder durch einen zweiwertigen aliphatischen Kohlenwasserstoffrest, der gegebenenfalls durch O unterbrochen ist, wie Niederalkylen oder Niederalkylenoxyniederalkylen, disubstituiert ist.

Substituiertes Amino ist beispielsweise durch einen aliphatischen oder araliphatischen Kohlenwasserstoffrest, wie Niederalkyl, Niederalkenyl, Niederalkinyl oder Phenyl-niederalkyl, -niederalkenyl oder -niederalkinyl, unabhängig voneinander mono-oder disubstituiertes oder durch einen zweiwertigen aliphatischen Kohlenwasserstoffrest, der gegebenenfalls durch O unterbrochen ist, wie Niederalkylen oder Niederalkylenoxyniederalkylen, disubstituiertes Amino. Als Beispiele seien Niederalkyl-, Niederalkenyl-, Niederalkinyl-, Phenylniederalkyl-, Phenylniederalkenyl-, Phenylniederalkinyl-, Diniederalkyl-, N-Niederalkyl-N-phenylniederalkyl- und Di(phenylniederalkyl)amino genannt.

Ein aliphatischer Kohlenwasserstoffrest ist beispielsweise Niederalkyl, Niederalkenyl oder in zweiter Linie Niederalkinyl.

Ein aliphatischer Kohlenwasserstoffrest, der durch O unterbrochen ist, ist insbesondere Niederalkoxy-niederalkyl, -niederalkenyl oder -niederalkinyl oder Niederalkenyloxy-niederalkyl, -niederalkenyl oder -niederalkinyl, während ein aliphatischer Kohlenwasserstoffrest, der durch $S(O)_m$ unterbrochen ist, insbesondere Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl ist.

Ein durch Halogen oder Hydroxy substituierter aliphatischer Kohlenwasserstoffrest ist beispielsweise Halogen-niederalkyl, -niederalkenyl oder -niederalkinyl oder Hydroxy-niederalkyl, -niederalkenyl oder -niederalkinyl.

Ein durch Halogen oder Hydroxy substituierter aliphatischer Kohlenwasserstoffrest, der durch O oder $S(O)_m$ unterbrochen ist, ist ein entsprechender vorstehend angegebener Rest, der durch Halogen oder Hydroxy substituiert ist.

Ein durch gegebenenfalls substituiertes Amino oder gegebenenfalls verestertes oder amidiertes Carboxy substituierter aliphatischer Kohlenwasserstoffrest, der gegebenenfalls durch O oder $S(O)_m$ unterbrochen ist, ist ein entsprechender vorstehend angegebener Rest, der durch Amino, vorstehend angegebenes substituiertes Amino, Carboxy, vorstehend angegebenes verestertes Carboxy oder vorstehend angegebenes amidiertes Carboxy substituiert ist.

Ein cycloaliphatischer Kohlenwasserstoffrest ist beispielsweise Cycloalkyl oder in zweiter Linie Cycloalkenyl.

Als araliphatische Kohlenwasserstoffreste kommen insbesondere Phenylniederalkyl, ferner Phenylniederalkenyl und -niederalkinyl in Frage.

Ein zweiwertiger aliphatischer Kohlenwasserstoffrest ist insbesondere Niederalkylen oder Niederalkenylen, wobei im Falle von alk das C-Atom, von dem die Doppelbindung ausgeht, insbesondere nicht mit dem N-Atom des Azabenzimidazolrings verknüpft ist; alk bedeutet in erster Linie Methylen.

Ein zweiwertiger aliphatischer Kohlenwasserstoffrest, der durch O unterbrochen ist, ist insbesondere Niederalkylenoxyniederalkylen.

Mit einem aliphatischen Alkohol verethertes Hydroxy ist insbesondere Niederalkoxy oder Niederalkenyloxy.

Vor- und nachstehend sind ungesättigte aliphatische, cycloaliphatische und araliphatische Substituenten in erster Linie nicht über ein C-Atom, von dem eine Mehrfachbindung ausgeht, mit einem aromatischen Rest verknüpft.

Phenyl bedeutet jeweils unsubstituiertes oder ein- oder mehrfach, z.B. zwei- oder dreifach, z.B. durch (einen) Substituenten ausgewählt aus der Gruppe bestehend aus Niederalkyl, Niederalkoxy, Halogen, Trifluormethyl und Hydroxy, substituiertes Phenyl.

Die Ringe A und B bilden einen Biphenylylrest, wobei entsprechendes 4-Biphenylyl bevorzugt ist.

Die vor- und nachstehend verwendeten Allgemeinbegriffe haben, sofern nicht abweichend definiert, folgende Bedeutungen:

Der Ausdruck "Nieder" bedeutet, dass entsprechende Gruppen und Verbindungen jeweils insbesondere bis und mit 7, vorzugsweise bis und mit 4, Kohlenstoffatome enthalten.

Halogen ist insbesondere Halogen mit einer Atomnummer bis und mit 35, d. h. Fluor, Chlor oder Brom, und umfasst ferner Iod.

Halogenalkansulfonylamino bedeutet insbesondere Halogen-$C_1$-$C_7$-alkansulfonylamino und ist z.B. Trifluormethan-, Difluormethan-, 1,1,2-Trifluorethan- oder Heptafluorpropan-sulfonylamino. Bevorzugt ist Halogen-$C_1$-$C_4$-alkansulfonylamino.

Niederalkanoyl bedeutet insbesondere $C_1$-$C_7$-Alkanoyl und ist z.B. Formyl, Acetyl, Propionyl, Butyryl, Isobutyryl oder Pivaloyl. Bevorzugt ist $C_2$-$C_5$-Alkanoyl.

Niederalkyl ist insbesondere $C_1$-$C_7$-Alkyl, d. h. Methyl, Ethyl, n-Propyl, Isopropyl, n-Butyl, Isobutyl, sek.-Butyl, tert.-Butyl oder ein entsprechender Pentyl-, Hexyl- oder Heptylrest. Bevorzugt ist $C_1$-$C_4$-Alkyl.

Niederalkenyl bedeutet insbesondere $C_3$-$C_7$-Alkenyl und ist z.B. Propen-2-yl, Allyl oder But-1-en-3-yl, -1-en-4-yl, -2-en-1-yl oder -2-en-2-yl. Bevorzugt ist $C_3$-$C_5$-Alkenyl.

Niederalkinyl ist insbesondere $C_3$-$C_7$-Alkinyl und bedeutet vorzugsweise Propargyl.

Niederalkoxy ist insbesondere $C_1$-$C_7$-Alkoxy d. h. Methoxy, Ethoxy, n-Propyloxy, Isopropyloxy, n-Butyloxy, Isobutyloxy, sek.-Butyloxy, tert.-Butyloxy oder entsprechendes Pentyloxy, Hexyloxy oder Heptyloxy. Bevorzugt ist $C_1$-$C_4$-Alkoxy.

Niederalkoxyniederalkyl bedeutet insbesondere $C_1$-$C_4$-Alkoxy-$C_1$-$C_4$-alkyl, wie 2-Methoxyethyl, 2-Ethoxyethyl, 2-(n-Propyloxy)ethyl oder Ethoxymethyl.

Niederalkoxy-niederalkenyl bzw. -niederalkinyl bedeutet insbesondere $C_1$-$C_4$-Alkoxy-$C_3$-$C_5$-alkenyl bzw. -alkinyl.

Niederalkoxycarbonyl bedeutet insbesondere $C_1$-$C_1$-Alkoxycarbonyl und ist z.B. Methoxy-, Ethoxy-, Propyloxy- oder Neopentyloxy-carbonyl. Bevorzugt ist $C_1$-$C_4$-Alkoxycarbonyl.

Niederalkenyloxy bedeutet insbesondere $C_3$-$C_7$-Alkenyloxy und ist z.B. Allyloxy, But-2-en-1-yloxy oder But-3-en-1-yloxy. Bevorzugt ist $C_3$-$C_5$-Alkenyloxy.

Niederalkenyloxycarbonyl bedeutet insbesondere $C_3$-$C_5$-Alkenyloxycarbonyl, vorzugsweise Allyloxycarbonyl, während Niederalkinyloxycarbonyl insbesondere $C_3$-$C_5$-Alkinyloxycarbonyl, wie Propargyloxycarbonyl, bedeutet.

Niederalkoxyniederalkoxycarbonyl bedeutet insbesondere $C_1$-$C_4$-Alkoxy-$C_1$-$C_4$-alkoxycarbonyl, vorzugsweise Ethoxyethoxycarbonyl, Methoxyethoxycarbonyl oder Isopropyloxyethoxycarbonyl.

Halogenniederalkyl bedeutet insbesondere Halogen-$C_1$-$C_4$-alkyl, wie Trifluormethyl, 1,1,2-Trifluor-2-chlor-ethyl, Chlormethyl oder n-Heptafluorpropyl.

Halogenniederalkenyl bedeutet insbesondere Halogen-$C_3$-$C_5$-alkenyl, wie 3-Chlorallyl.

Halogenniederalkinyl ist insbesondere Halogen-$C_3$-$C_5$-alkinyl, wie 3-Chlorpropargyl.

Hydroxyniederalkyl bedeutet insbesondere Hydroxy-$C_1$-$C_4$-alkyl, wie Hydroxymethyl, 2-Hydroxyethyl oder 3-Hydroxypropyl.

Hydroxyniederalkenyl bedeutet insbesondere Hydroxy-$C_3$-$C_5$-alkenyl, wie 3-Hydroxyallyl.

Hydroxyniederalkinyl bedeutet insbesondere Hydroxy-$C_3$-$C_5$-alkinyl, wie 3-Hydroxypropargyl.

Phenylniederalkyl ist insbesondere Phenyl-$C_1$-$C_4$-alkyl und bedeutet vorzugsweise Benzyl oder 1- oder 2-Phenethyl, während Phenylniederalkenyl bzw. Phenylniederalkinyl insbesondere Phenyl-$C_3$-$C_5$-alkenyl bzw. -alkinyl bedeuten, insbesondere 3-Phenylallyl oder 3-Phenylpropargyl.

Niederalkylen bedeutet insbesondere $C_2$-$C_7$-Alkylen, ist geradkettig oder verzweigt und bedeutet insbesondere Ethylen, 1,3-Propylen, 1,4-Butylen, 1,2-Propylen, 2-Methyl-1,3-propylen oder 2,2-Dimethyl-1,3-propylen. Bevorzugt ist $C_2$-$C_5$-Alkylen.

Niederalkylenamino bedeutet insbesondere $C_2$-$C_7$-Alkylenamino, ist geradkettig oder verzweigt und bedeutet insbesondere Ethylenamino, 1,3-Propylenamino, 1,4-Butylenamino, 1,2-Propylenamino, 2-Methyl-1,3-propylenamino oder 2,2-Dimethyl-1,3-propylenamino. Bevorzugt ist $C_2$-$C_5$-Alkylenamino.

Niederalkylenoxyniederalkylen bedeutet insbesondere $C_2$-$C_4$-Alkylenoxy-$C_2$-$C_4$-alkylen, vorzugsweise Ethylenoxyethylen.

Niederalkylenoxyniederalkylenamino bedeutet insbesondere $C_2$-$C_4$-Alkylenoxy-$C_2$-$C_4$-alkylenamino, vorzugsweise Ethylenoxyethylenamino.

Niederalkylamino bedeutet insbesondere $C_1$-$C_7$-Alkylamino und ist z.B. Methyl-, Ethyl-, n-Propyl- oder Isopropyl-amino. Bevorzugt ist $C_1$-$C_4$-Alkylamino.

Niederalkenylamino bedeutet vorzugsweise $C_3$-$C_5$-Alkenylamino, wie Allyl- oder Methallyl-amino.

Niederalkinylamino bedeutet vorzugsweise $C_3$-$C_5$-Alkinylamino, wie Propargylamino.

Phenylniederalkylamino bedeutet vorzugsweise Phenyl-$C_1$-$C_4$-alkylamino, insbesondere Benzyl- oder 1-

oder 2-Phenylethyl-amino.

Phenylniederalkenylamino bedeutet vorzugsweise Phenyl-$C_3$-$C_5$-alkenylamino, insbesondere Phenylallylamino oder 3-Phenylmethallylamino.

Phenylniederalkinylamino bedeutet vorzugsweise Phenyl-$C_3$-$C_5$-alkinylamino, insbesondere Phenylpropargylamino.

Diniederalkylamino bedeutet insbesondere Di-$C_1$-$C_4$-alkylamino, wie Dimethyl-, Diethyl-, Di(n-propyl)-, Methyl-propyl-, Methyl-ethyl-, Methyl-butyl- oder Dibutyl-amino.

N-Niederalkyl-N-phenylniederalkyl-amino bedeutet insbesondere N-$C_1$-$C_4$-Alkyl-N-phenyl-$C_1$-$C_4$-alkylamino, vorzugsweise Methyl-benzyl-amino oder Ethyl-benzyl-amino.

Di(phenylniederalkyl)amino bedeutet insbesondere Di(phenyl-$C_1$-$C_4$-alkyl)amino, vorzugsweise Dibenzylamino.

Niederalkenyloxyniederalkyl bedeutet insbesondere $C_3$-$C_5$-Alkenyloxy-$C_1$-$C_4$-alkyl, wie 2-Allyloxyethyl, und Niederalkenyloxy-niederalkenyl bzw. -niederalkinyl bedeutet insbesondere $C_3$-$C_5$-Alkenyloxy-$C_3$-$C_5$-alkenyl bzw. -alkinyl.

Niederalkylthio-niederalkenyl bzw. -niederalkinyl bedeutet insbesondere $C_1$-$C_4$-Alkylthio-$C_3$-$C_5$-alkenyl bzw. -alkinyl.

Niederalkylthioniederalkyl bedeutet insbesondere $C_1$-$C_4$-Alkylthio-$C_1$-$C_4$-alkyl, wie Ethylthiomethyl, 2-Ethylthioethyl, 2-Methylthioethyl oder 2-Isopropylthioethyl, während als Niederalkan-sulfinylniederalkyl bzw. -sulfonylniederalkyl insbesondere entsprechende $C_1$-$C_4$-Alkan-sulfinyl-$C_1$-$C_4$-alkylreste bzw. -sulfonyl-$C_1$-$C_4$-alkylreste in Frage kommen.

Niederalkenylthioniederalkyl ist insbesondere $C_3$-$C_5$-Alkenylthio-$C_1$-$C_4$-alkyl, wie 1-Allylthioethyl or 3-Allylthiopropyl, waehrend Niederalkenyl-sulfinylniederalkyl bzw. -sulfonylniederalkyl insbesondere $C_3$-$C_5$-Alkenyl-sulfinyl-$C_1$-$C_4$-alkyl bzw. -sulfonyl-$C_1$-$C_4$-alkyl bedeutet.

Niederalkinylthioniederalkyl ist insbesondere $C_3$-$C_5$-Alkinylthio-$C_1$-$C_4$-alkyl, wie 2-Propargylthioethyl oder 3-Propargylthiopropyl, während Niederalkinyl-sulfinylniederalkyl bzw. -sulfonylniederalkyl insbesondere $C_3$-$C_5$-Alkinyl-sulfinyl-$C_1$-$C_4$-alkyl bzw. -sulfonyl-$C_1$-$C_4$-alkyl bedeutet.

Cycloalkyl ist insbesondere $C_3$-$C_7$-Cycloalkyl, d. h. Cyclopropyl, Cyclobutyl, Cyclopentyl, Cyclohexyl oder Cycloheptyl. Bevorzugt sind Cyclopentyl und Cyclohexyl.

Cycloalkenyl ist insbesondere $C_3$-$C_7$-Cycloalkenyl und bedeutet vorzugsweise Cyclopent-2-enyl oder -3-enyl oder Cyclohex-2-enyl oder -3-enyl.

Niederalkenylen ist insbesondere $C_3$-$C_5$-Alkenylen und bedeutet z.B. But-2-en-1,4-ylen.

Niederalkoxynieder-alkenyloxycarbonyl bzw. -alkinyloxycarbonyl ist insbesondere $C_1$-$C_4$-Alkoxy-$C_3$-$C_5$-alkenyloxycarbonyl bzw. -alkinyloxycarbonyl.

Ausgedehnte pharmakologische Untersuchungen haben ergeben, dass die Verbindungen I und ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser-Retention. Die Folge der Angiotensin-II-Aktivität manifestiert sich unter anderem in einer Erhöhung des Blutdrucks.

Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretion-stimulierenden Effekte zu unterdrücken.

Die Angiotensin-II-antagonisierenden Eigenschaften der Verbindungen der Formel I und ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7,4) unter Einsatz von Peptidaseinhibitoren suspendiert. Die Proben werden 60 Minuten bei 25 °C mit $^{125}$I-Angiotensin-II (0,175 nM) und einer variierenden Konzentration an Angiotensin-II oder an Testsubstanz inkubiert. Die Inkubation wird dann durch Zugabe von mit eiskaltem Phosphat gepuffertem Kochsalz beendet und es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die Verbindungen der Formel I und ihre pharmazeutisch verwendbaren Salze werden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammern bei einer anfänglich bestehenden Spannung von 2 g fixiert. Anschliessend werden die Ringe bei 37 °C in 20 ml eines Gewebebades getaucht und mit einem Gemisch aus 95 % $O_2$ und 5 % $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der

Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die Verbindungen der Formel I und ihre pharmazeutisch verwendbaren Salze werden $IC_{50}$-Werte ab etwa 5 nM bestimmt.

Dass die Verbindungen der Formel I und ihre pharmazeutisch verwendbaren Salze durch Angiotensin-II induzierten Bluthochdruck reduzieren können, kann im Testmodell der normotensiven, narkotisierten Ratte verifiziert werden. Nach Kalibration der Präparationen mit jeweils 0,9 % NaCl (1 ml/kg i.v.), Noradrenalin (1 µg/kg i.v.) bzw. Angiotensin-II (0,3 µg/kg i.v.) werden steigende Dosen (3-6) der Testsubstanz durch Bolusinjektion intravenös injiziert, worauf nach jeder Dosis in 5 Minuten-Intervallen Angiotensin-II bzw. Noradrenalin appliziert wird. Der Blutdruck wird direkt in der Halsschlagader gemessen und mit einem on-line Datenerfassungssystem aufgezeichnet (Buxco). Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen der Formel I und ihre pharmazeutisch verwendbaren Salze ab einer Dosis von etwa 0,3 mg/kg i.v. einen hemmenden Effekt.

Auch im Testmodell der renalen hypertensiven Ratte kann die antihypertensive Aktivität der Verbindungen der Formel I und ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [ Helv. Physiol. Acta 24 (1966), 58] vor Verabreichung der Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen der Formel I und ihre pharmazeutisch verwendbaren Salze z.B. als Wirkstoffe in Antihypertensiva verwendet werden, welche z.B. zur Behandlung von Bluthochdruck sowie von Herzinsuffizienz eingesetzt werden. Ein Erfindungsgegenstand ist somit die Verwendung der Verbindungen der Formel I und ihrer pharmazeutisch verwendbaren Salze zur Herstellung von entsprechenden Arzneimitteln und zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Die Erfindung betrifft insbesondere Verbindungen der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, oder 5-Tetrazolyl bedeutet oder R für -Z-R′ steht, worin Z für eine Bindung oder für O,S(O)$_m$ oder NH steht, R′ Wasserstoff oder jeweils gegebenenfalls durch Halogen, durch Hydroxy, durch Amino, welches gegebenenfalls wie unmittelbar vorstehend in der Definition der Aminogruppe des Carbamoylrestes R angegeben substituiert ist, durch Carboxy, welches gegebenenfalls wie unmittelbar vorstehend angegeben verestert ist, oder durch Carbamoyl, in welchem die Aminogruppe gegebenenfalls wie unmittelbar vorstehend angegeben substituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkylthioniederalkyl, Niederalkylthioniederalkenyl, Niederalkylthioniederalkinyl, Niederalkansulfinylniederalkyl, Niederalkansulfonylniederalkyl, Niederalkenylthioniederalkyl, Niederalkenylsulfinylniederalkyl, Niederalkenylsulfonylniederalkyl, Niederalkinylthioniederalkyl, Niederalkinylsulfinylniederalkyl oder Niederalkinylsulfonylniederalkyl bedeutet und der Index m für 0, 1 oder 2 steht, $R_1$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet und $R_2$ für die Gruppe der Formel Ia steht, in der alk Niederalkylen oder Niederalkenylen bedeutet, $R_3$ COOH, $SO_3H$, Halogenniederalkansulfonylamino, $PO_2H_2$, $PO_3H_2$ oder 5-Tetrazolyl bedeutet und entweder die Ringe A und B unabhängig voneinander gegebenenfalls durch Halogen, durch jeweils gegebenenfalls durch Halogen oder Hydroxy substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl oder Niederalkenyloxyniederalkinyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, substituiert sind oder der Ring A durch 5-Tetrazolyl substituiert ist und der Ring B gegebenenfalls wie unmittelbar vorstehend angegeben substituiert ist, in freier Form oder in Salzform.

Die Erfindung betrifft insbesondere Verbindungen der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, Niederalkoxycarbonyl, Niederalkoxyniederalkoxycarbonyl, Carbamoyl, in welchem die Aminogruppe gegebenenfalls durch Niederalkyl oder Phenylniederalkyl unabhängig voneinander mono- oder disubstituiert oder durch Niederalkylen disubstituiert ist, oder R für $-Z-R'$ steht, worin Z für eine Bindung oder für O steht und $R'$ Wasserstoff oder jeweils gegebenenfalls durch Halogen, durch Hydroxy, durch Amino, welches gegebenenfalls durch Niederalkyl oder Phenylniederalkyl unabhaengig voneinander mono- oder disubstituiert oder durch Niederalkylen disubstituiert ist, durch Carboxy, durch Niederalkoxycarbonyl oder durch Niederalkoxyniederalkoxycarbonyl substituiertes Niederalkyl oder Niederalkoxyniederalkyl bedeutet, $R_1$ jeweils gegebenenfalls durch Halogen oder Hydroxy substituiertes Niederalkyl oder Niederalkenyl, 3- bis 7-gliedriges Cycloalkyl oder Phenylniederalkyl bedeutet und $R_2$ für die Gruppe der Formel Ia steht, in der alk Niederalkylen bedeutet, $R_3$ COOH oder 5-Tetrazolyl bedeutet und die Ringe A und B unabhängig voneinander gegebenenfalls durch Halogen, durch gegebenenfalls durch Halogen oder Hydroxy substituiertes Niederalkyl, durch Niederalkoxy, durch Carboxy oder durch Niederalkoxycarbonyl substituiert sind, in freier Form oder in Salzform.

Die Erfindung betrifft insbesondere Verbindungen der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, Niederalkoxycarbonyl, gegebenenfalls durch Niederalkyl mono- oder disubstituiertes Carbamoyl oder 5-Tetrazolyl bedeutet oder R für $-Z-R'$ steht, worin Z für eine Bindung oder für O, $S(O)_m$ oder NH steht, m für 0, 1 oder 2 steht und $R'$ Wasserstoff oder gegebenenfalls durch Halogen, Hydroxy oder Amino substituiertes Niederalkyl bedeutet, $R_1$ Niederalkyl, Niederalkenyl, Hydroxyniederalkyl, Halogenniederalkyl, 3- bis 7-gliedriges Cycloalkyl oder Phenylniederalkyl bedeutet und $R_2$ für die Gruppe der Formel Ia steht, in der alk Niederalkylen bedeutet, $R_3$ COOH oder 5-Tetrazolyl bedeutet und die Ringe A und B unabhängig voneinander gegebenenfalls durch Halogen, durch gegebenenfalls durch Halogen oder Hydroxy substituiertes Niederalkyl, durch Niederalkoxy, durch Carboxy oder durch Niederalkoxycarbonyl substituiert sind, in freier Form oder in Salzform.

Die Erfindung betrifft insbesondere Verbindungen der Formel I, worin $R_2$ für die Gruppe der Formel

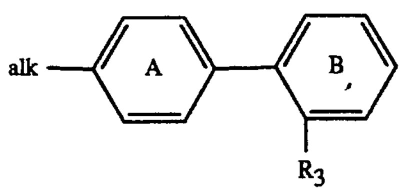

(Ib)

steht, in freier Form oder in Salzform.

Die Erfindung betrifft insbesondere Verbindungen der Formel I, worin eine oder zwei der Variablen $Z_1$, $Z_2$, $Z_3$ und $Z_4$ für N und die anderen für C(R) stehen, wobei R Wasserstoff, Halogen, Carboxy, Niederalkoxycarbonyl, Niederalkyl, Halogenniederalkyl, Hydroxyniederalkyl oder Niederalkoxy bedeutet, $R_1$ Niederalkyl, Niederalkenyl, Hydroxyniederalkyl, Halogenniederalkyl, 3- bis 7-gliedriges Cycloalkyl oder Phenylniederalkyl bedeutet und $R_2$ für die Gruppe der Formel Ib steht, in der alk Niederalkylen bedeutet, $R_3$ COOH oder 5-Tetrazolyl bedeutet und entweder die Ringe A und B unabhängig voneinander gegebenenfalls durch Halogen, Niederalkyl, Halogenniederalkyl, Niederalkoxy, Carboxy oder Niederalkoxycarbonyl substituiert sind oder der Ring A durch 5-Tetrazolyl substituiert ist und der Ring B gegebenenfalls wie unmittelbar vorstehend angegeben substituiert ist, in freier Form oder in Salzform.

Die Erfindung betrifft insbesondere Verbindungen der Formel I, worin $R_2$ fuer die Gruppe der Formel Ia oder Ib steht und alk Methylen bedeutet, in freier Form oder in Salzform.

Die Erfindung betrifft in erster Linie Verbindungen der Formel I, worin eine oder zwei der Variablen $Z_1$, $Z_2$, $Z_3$ und $Z_4$ für N und die anderen für CH stehen, insbesondere stehen $Z_1$, $Z_2$ und $Z_3$ für CH und $Z_4$ für N, $R_1$ Niederalkyl, insbesondere mit bis und mit 4 C-Atomen, wie Propyl oder n-Butyl, Niederalkenyl, insbesondere mit 3 bis und mit 5 C-Atomen, wie Propen-2-yl oder But-2-en-1-yl, oder Halogenniederalkyl, insbesondere mit bis und mit 4 C-Atomen und Halogen mit einer Atomnummer bis und mit 35, wie n-

Heptafluorpropyl, bedeutet und $R_2$ für die Gruppe der Formel Ib steht, in der alk Methylen bedeutet, $R_3$ COOH oder 5-Tetrazolyl ist und die Ringe A und B unabhängig voneinander unsubstituiert oder in zweiter Linie durch Halogen, insbesondere mit einer Atomnummer bis und mit 35, wie Chlor, Niederalkyl, insbesondere mit bis und mit 4 C-Atomen, wie Methyl, oder Niederalkoxy, insbesondere mit bis und mit 4 C-Atomen, wie Methoxy, substituiert sind, in freier Form oder in Salzform.

Die Erfindung betrifft insbesondere Verbindungen der Formel I, worin eine oder zwei der Variablen $Z_1$, $Z_2$, $Z_3$ und $Z_4$ für N und die anderen für CH stehen oder worin insbesondere eine der Variablen $Z_2$, $Z_3$ und $Z_4$ fuer N und $Z_1$ und die anderen der Variablen $Z_2$, $Z_3$ und $Z_4$ fuer C(R), insbesondere CH, stehen oder worin $Z_1$ und $Z_3$ fuer C(R), insbesondere CH, und $Z_2$ und $Z_4$ fuer N stehen, in freier Form oder in Salzform.

Die Erfindung betrifft in erster Linie Verbindungen der Formel I, worin $Z_1$, $Z_2$ und $Z_3$ für CH und $Z_4$ für N oder $Z_1$ und $Z_3$ fuer CH und $Z_2$ und $Z_4$ fuer N stehen, $R_1$ Niederalkyl, insbesondere mit bis und mit 4 C-Atomen, wie Propyl oder n-Butyl, Niederalkenyl, insbesondere mit 3 bis und mit 5 C-Atomen, wie Propen-2-yl oder But-2-en- 1-yl, oder Halogenniederalkyl, insbesondere mit bis und mit 4 C-Atomen und Halogen mit einer Atomnummer bis und mit 35, wie n-Heptafluorpropyl, bedeutet und $R_2$ für die Gruppe der Formel Ib steht, in der alk Methylen bedeutet, $R_3$ COOH oder 5-Tetrazolyl ist und die Ringe A und B unabhängig voneinander unsubstituiert oder in zweiter Linie durch Halogen, insbesondere mit einer Atomnummer bis und mit 35, wie Chlor, Niederalkyl, insbesondere mit bis und mit 4 C-Atomen, wie Methyl, oder Niederalkoxy, insbesondere mit bis und mit 4 C-Atomen, wie Methoxy, substituiert sind, in freier Form oder in Salzform.

Die Erfindung betrifft in erster Linie Verbindungen der Formel I, worin $Z_1$, $Z_2$ und $Z_3$ für CH stehen, $Z_4$ für N steht, $R_1$ $C_3$-$C_4$-Alkyl, wie Propyl oder n-Butyl, bedeutet und $R_2$ für die Gruppe der Formel Ib steht, in der alk Methylen bedeutet, $R_3$ 5-Tetrazolyl bedeutet und die Ringe A und B unsubstituiert sind, in freier Form oder in Salzform.

Die Erfindung betrifft namentlich die in den Beispielen genannten neuen Verbindungen der Formel I in freier Form oder in Salzform.

Ein weiterer Gegenstand der Erfindung ist ein Verfahren zur Herstellung der Verbindungen der Formel I und ihrer Salze, z.B. dadurch gekennzeichnet, dass man

a) eine Verbindung der Formel

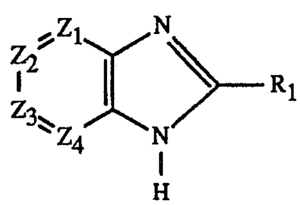

(IIa)

oder ein Salz davon mit einer Verbindung der Formel

$X_1$-$R_2$ (IIb)

oder einem Salz davon, worin $X_1$ reaktionsfähiges verestertes Hydroxy bedeutet, umsetzt oder

b) in einer Verbindung der Formel

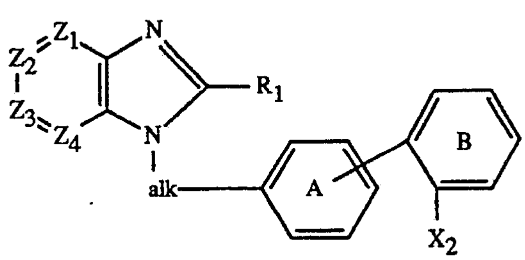

(III)

oder einem Salz davon, worin $X_2$ einen in die Variable $R_3$ überführbaren Rest bedeutet, $X_2$ in die Variable $R_3$ überführt oder

c) eine Verbindung der Formel

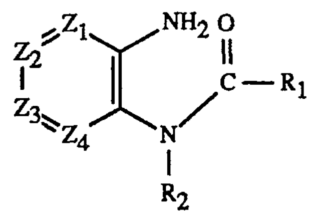

(IV)

oder ein Salz davon cyclisiert und jeweils, wenn erwünscht, eine verfahrensgemäss oder auf andere Weise erhältliche Verbindung der Formel I in freier Form oder in Salzform in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein Salz oder ein verfahrensgemäss erhältliches Salz einer Verbindung dr Formel I in die freie Verbindung der Formel I oder in ein anderes Salz überführt.

Salze von Ausgangsmaterialien, die mindestens ein basisches Zentrum aufweisen, sind entsprechende Säureadditionssalze, während Salze von Ausgangsstoffen, die mindestens eine acide Gruppe aufweisen, Salze mit Basen sind, jeweils wie in Zusammenhang mit entsprechenden Salzen von Verbindungen der Formel I vorstehend aufgeführt.

Reaktionsfähiges verestertes Hydroxy $X_1$ ist insbesondere mit einer starken anorganischen Säure oder organischen Sulfonsäure verestertes Hydroxy, beispielsweise Halogen, wie Chlor, Brom oder Iod, oder Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Bromphenyl-oder p-Toluol-sulfonyloxy.

In die Variable $R_3$ überführbare Reste $X_2$ sind beispielsweise Cyano, Mercapto, Halogen, die Gruppe $-N_2^+A^-$, in der $A^-$ ein von einer Säure abgeleitetes Anion bedeutet, Amino, funktionelle Derivate von COOH, $SO_3H$, $PO_3H_2$ und $PO_2H_2$ und N-geschütztes 5-Tetrazolyl.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80° C bis zur Siedetemperatur des Reaktionsmediums, vorzugsweise von etwa -10° bis etwa +200° C, und, falls erforderlich, in einem geschlossenen Gefäss, unter Druck, in einer Inertgasatmosphäre und/oder unter wasserfreien Bedingungen arbeitet.

Variante a):

Die Umsetzung von Verbindungen der Formel IIa mit Verbindungen der Formel IIb erfolgt vorteilhaft in Gegenwart einer Base. Als Basen kommen insbesondere Alkalimetall-hydroxide, -hydride, -amide, -alkanolate, -carbonate, -triphenylmethylide, -diniederalkylamide, -aminoalkylamide oder -niederalkylsilylamide, Naphthylamine, Diund Tri-niederalkylamine, basische Heterocyclen, Ammoniumhydroxide, sowie carbocyclische Amine in Betracht. Beispielhaft seien Natrium-hydroxid, -hydrid, -amid, -(m)-ethylat, Kalium-tert.-butylat, -carbonat, Lithium-triphenylmethylid, -diisopropylamid, Dimethylaminonaphthalin, Di- und Triethylamin, Diisopropyl-ethyl-amin, N-Methylpiperidin, Pyridin, Benzyltrimethylammoniumhydroxid, 1,5-Diazabicyclo[4.3.0]non-5-en (DBN) und 1,8-Diazabicyclo[5.4.0]undec-7-en (DBU) genannt

$X_1$ bedeutet vorzugsweise Halogen, wie Chlor oder Brom, oder Sulfonyloxy, wie $C_1$-$C_7$-Alkan-, z.B. Methan-sulfonyloxy, oder gegebenenfalls, z.B. durch $C_1$-$C_7$-Alkyl, substituiertes Benzolsulfonyloxy, z.B. p-Toluolsulfonyloxy.

Zur Herstellung der Ausgangsverbindungen der Formel IIa geht man in an sich bekannter Weise beispielsweise von Verbindungen der Formel

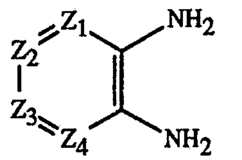

(IIc)

aus und setzt diese unter Erwärmen mit Verbindungen der Formel $R_1$-COOH (IId) um.

Das Ausgangsmaterial der Formel IIb ist bekannt oder kann in an sich bekannter Weise hergestellt werden.

Variante b):

In 5-Tetrazolyl $R_3$ überführbare Reste $X_2$ sind beispielsweise Cyano und N-geschütztes 5-Tetrazolyl.

Zur Herstellung von Verbindungen der Formel I, worin $R_3$ 5-Tetrazolyl bedeutet, geht man beispielsweise von Ausgangsmaterial der Formel III aus, worin $X_2$ Cyano bedeutet, und setzt dieses mit einem Azid, z. B. mit $HN_3$ oder insbesondere einem Salz, wie Alkalimetallsalz, davon oder mit einem Organozinnazid, wie Triniederalkyl- oder Triarylzinnazid, um. Bevorzugte Azide sind beispielsweise Natrium- und Kaliumazid sowie Tri-$C_1$-$C_4$-alkyl-, z.B. Triethyl- oder Tributyl-zinnazid, und Triphenylzinnazid.

Als Schutzgruppen von N-geschütztem 5-Tetrazolyl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- oder Ethoxymethyl, Niederalkylthiomethyl, wie Methylthiomethyl, sowie 2-Cyanoethyl, ferner Niederalkoxyniederalkoxymethyl, wie 2-Methoxyethoxymethyl, Benzyloxymethyl sowie Phenacyl. Die Abspaltung der Schutzgruppen erfolgt in Anlehnung an bekannte Methoden. So wird z.B. Triphenylmethyl üblicherweise durch Hydrolyse, insbesondere in Gegenwart einer Säure, oder Hydrogenolyse in Gegenwart eines Hydrierungskatalysators, 4-Nitrobenzyl z.B. durch Hydrogenolyse in Gegenwart eines Hydrierungskatalysators, Methoxy- oder Ethoxymethyl z.B. durch Behandeln mit einem Triniederalkyl-, wie Triethyl- oder Tributyl-zinn-bromid, Methylthiomethyl z.B. durch Behandeln mit Trifluoressigsäure, 2-Cyanoethyl z.B. durch Hydrolyse, beispielsweise mit Natronlauge, 2-Methoxyethoxymethyl z.B. durch Hydrolyse, z.B. mit Salzsäure, und Benzyloxymethyl und Phenacyl z.B. durch Hydrogenolyse in Gegenwart eines Hydrierungskatalysators abgespalten.

Ein in $SO_3H$ $R_3$ überführbarer Rest $X_2$ ist beispielsweise die Mercaptogruppe. Eine solche Gruppe aufweisende Ausgangsverbindungen der Formel III werden beispielsweise durch an sich bekannte Oxidationsverfahren zu solchen Verbindungen der Formel I oxidiert, worin $R_3$ $SO_3H$ ist. Als Oxidationsmittel kommen beispielsweise anorganische Persäuren, wie Persäuren von Mineralsäuren, z.B. Periodsäure oder Perschwefelsäure, organische Persäuren, wie Percarbon- oder Persulfon-säuren, z.B. Perameisen-, Peressig-, Trifluorperessig- oder Perbenzoe-säure oder p-Toluolpersulfonsäure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolframoxid, zu nennen sind. Die Oxidation wird unter milden Bedingungen, z.B. bei Temperaturen von etwa -50° bis etwa + 100° C, durchgeführt.

Unter einer in $PO_3H_2$ $R_3$ überführbaren Gruppe ist beispielsweise eine Gruppe $-N_2^+ A^-$ zu verstehen, wobei $A^-$ für ein Anion einer Säure, wie Mineralsäure, steht. Entsprechende Diazoniumverbindungen werden beispielsweise in an sich bekannter Weise mit einem P(III)-Halogenid, wie $PCl_3$ oder $PBr_3$, umgesetzt und hydrolytisch aufgearbeitet, wobei solche Verbindungen der Formel I erhältlich sind, worin $R_3$ $PO_3H_2$ ist.

Verbindungen I, worin $R_3$ $PO_2H_2$ ist, erhält man z. B. durch in üblicher Weise erfolgende Umwandlung von $X_2$ in einer Verbindung III, worin $X_2$ ein funktionelles Derivat von $PO_2H_2$ ist, in $PO_2H_2$.

Als in Halogenalkansulfonylamino $R_3$ überführbarer Rest $X_2$ kommt beispielsweise Amino in Frage. Zur Herstellung von Verbindungen der Formel I, worin $R_3$ Halogenalkansulfonylamino bedeutet, setzt man beispielsweise entsprechende Aniline mit einer üblicherweise reaktionsfähig veresterten Halogenalkansulfonsäure um, wobei gegebenenfalls in Gegenwart einer Base gearbeitet wird. Die bevorzugte reaktionsfähig veresterte Halogenalkansulfonsäure ist das entsprechende Halogenid, wie Chlorid oder Bromid.

Ein in COOH $R_3$ überführbarer Rest $X_2$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, n-Propanol, Isopropanol, n-Butanol, sek.-Butanol oder tert.-Butanol, während als cycloaliphatischer Alkohol beispielsweise ein 3- bis 8-gliedriges Cycloalkanol, wie Cyclopentanol, -hexanol oder -heptanol, in Frage kommt. Ein aromatischer Alkohol ist beispielsweise ein Phenol oder ein heterocyclischer Alkohol, welche jeweils substituiert sein können, insbesondere Hydroxypyridin, z.B. 2-, 3- oder 4-Hydroxypyridin.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen oder 3-Aza-, 3-Niederalkylaza-, 3-Oxa- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- und N,N-Dipropyl-carbamoyl, Pyrrolidino- und Piperidinocarbonyl, Morpholino-, Piperazino-, 4-Methylpiperazino- und Thiomorpholino-carbonyl, Anilinocarbonyl und durch Niederalkyl, Niederalkoxy und/oder Halogen substituiertes Anilinocarbonyl zu nennen.

Bevorzugtes funktionell abgewandeltes Carboxy ist beispielsweise Niederalkoxycarbonyl, wie Methoxy- oder Ethoxycarbonyl, und Cyano.

Verbindungen der Formel I, worin $R_3$ Carboxy ist, können beispielsweise ausgehend von Verbindungen der Formel III, worin $X_2$ Cyano oder verestertes oder amidiertes Carboxy bedeutet, durch Hydrolyse, insbesondere in Gegenwart einer Base, oder, ausgehend von Verbindungen der Formel III, worin $X_2$ Hydroxymethyl oder Formyl bedeutet, durch Oxidation hergestellt werden. Die Oxidation erfolgt beispielsweise in einem inerten Lösungsmittel, wie in einer Niederalkancarbonsäure, z.B. Essigsäure, einem Keton, z.B. Aceton, einem Ether, z.B. Tetrahydrofuran, einem heterocyclischen Aromaten, z.B. Pyridin, oder in Wasser, oder in einem Gemisch davon, erforderlichenfalls unter Kühlen oder Erwärmen, z.B. in einem Temperaturbereich von etwa 0° bis etwa +150° C. Als Oxidationsmittel kommen beispielsweise oxidierende Übergangsmetallverbindungen, insbesondere solche mit Elementen der I., VI. oder VII. Nebengruppe, in Frage. Als Beispiele seien genannt: Silberverbindungen, wie Silber-nitrat, -oxid und -picolinat, Chromverbindungen, wie Chromtrioxid und Kaliumdichromat, und Manganverbindungen, wie Kalium-, Tetrabutylammonium- und Benzyltriethylammonium-permanganat. Weitere Oxidationsmittel sind beispielsweise geeignete Verbindungen mit Elementen der IV. Hauptgruppe, wie Bleidioxid, oder Halogen-Sauerstoff-Verbindungen, wie Natriumiodat oder Kaliumperiodat.

Vorzugsweise eignet sich die Variante b) zur Herstellung solcher Verbindungen der Formel I, worin die Variablen Bedeutungen haben, die von ungesättigten Resten verschieden sind.

Das Ausgangsmaterial der Formel III ist beispielsweise zugänglich, indem man von Verbindungen der Formel IIa ausgeht und diese in Analogie zur Variante a) mit einer Verbindung der Formel

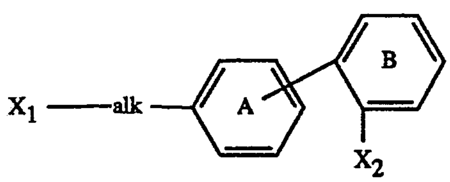

(IIIa),

worin $X_1$ und $X_2$ die vorstehend aufgeführten Bedeutungen haben, umsetzt.

Verbindungen der Formel IIIa sind bekannt oder können nach an sich bekannten Methoden hergestellt werden.

Variante c):

Diese Variante eignet sich insbesondere für die Herstellung solcher Verbindungen der Formel I, worin $R_3$ 5-Tetrazolyl bedeutet.

Die Cyclisierung wird in an sich bekannter Weise durchgeführt, beispielsweise unter Erwärmen, z.B. in einem Temperaturbereich von etwa 70° bis etwa 200° C, vorzugsweise bei Rückflusstemperatur des Lösungsmittelsystems, gegebenenfalls in Gegenwart einer Säure, wie einer Mineral- oder Carbonsäure, z.B. von Essigsäure.

Das Ausgangsmaterial der Formel IV ist unter Anwendung üblicher Methoden zugänglich, beispielsweise durch Umsetzung einer Verbindung der Formel

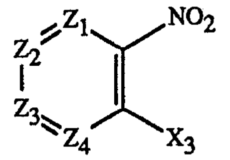

(IVa),

worin $X_3$ Halogen, wie Chlor, bedeutet, mit einer Verbindung der Formel $H_2N-R_2$ (IVb), an die sich im nächsten Reaktionsschritt eine gegebenenfalls durch Basen katalysierte N-Acylierung mit einer Verbindung der Formel $HOOC-R_1$ (IId) oder einem reaktionsfähigen Säurederivat, insbesondere einem Säurehalogenid, davon, anschliesst. Die so erhältlichen Verbindungen der Formel

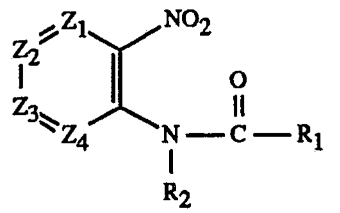

(IVc)

können anschliessend, beispielsweise durch Hydrierung, zu den entsprechenden Verbindungen der Formel IV reduziert werden.

Eine verfahrensgemäss oder auf andere Weise erhältliche Verbindung der Formel I kann in an sich bekannter Weise in eine andere Verbindung der Formel I überführt werden.

Eine Hydroxy aufweisende Verbindung der Formel I kann z. B. nach an sich bekannten Methoden verethert werden. Die Veretherung kann z.B. mit einem Alkohol, wie einem gegebenenfalls substituierten Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide oder Methan-, Benzol- oder p-Toluol-sulfonate, in Betracht. Die Veretherung kann z.B. in Gegenwart einer Base, z. B. eines Alkalimetall-hydrids, -hydroxids oder -carbonats oder eines basischen Amins, erfolgen. Umgekehrt können entsprechende Ether, wie Niederalkoxyverbindungen, z.B. mittels starker Säuren, wie Mineralsäuren, z.B. Brom- oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa +100°C, in An- oder Abwesenheit eines Lösungs- oder Verdünnungsmittels, unter Inertgas und/oder unter Druck und gegebenenfalls in einem geschlossenen Gefäss durchgeführt werden.

Falls ein aromatischer Strukturbestandteil durch Niederalkylthio substituiert ist, kann man dieses auf übliche Weise zu entsprechendem Niederalkan-sulfinyl oder -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie Percarbon- oder Persulfon säuren, z.B. Perameisen-, Peressig-, Trifluorperessig-, Perbenzoe- oder p-Toluolpersulfon-säure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa +100°C, durchgeführt Die weitere Oxidation zur Sulfonstufe kann man mit Distickstofftetroxid als Katalysator in Gegenwart von Sauerstoff bei tiefen Temperaturen entsprechend durchführen, ebenso wie die direkte Oxidation von Niederalkylthio zu Niederalkansulfonyl. Jedoch setzt man hierbei üblicherweise das Oxidationsmittel im Überschuss ein.

Weist eine der Variablen Amino auf, können entsprechende Verbindungen [in an sich bekannter Weise N-(ar)alkyliert werden; ebenso können Carbamoyl bzw. Carbamoyl aufweisende Reste N-(ar)alkyliert werden. Die (Ar-)Alkylierung erfolgt z.B. mit einem (Aryl-)$C_1$-$C_7$-Alkyl-halogenid, z.B. -bromid oder -iodid, (Aryl-)$C_1$-$C_7$-Alkansulfonat, z.B. mit Methansulfonat oder p-Toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft in Gegenwart eines Phasentransfer-Katalysators, wie von Tetrabutylammoniumbromid oder Benzyltrimethylammoniumchlorid, wobei indes stärker basische Kondensationsmittel, wie Alkalimetall-amide, -hydride oder -alkoholate, z.B. Natriumamid, Natriumhydrid oder Natriumethanolat, erforderlich sein können.

In Verbindungen der Formel I, die als Substituenten eine veresterte oder amidierte Carboxygruppe aufweisen, kann man eine solche Gruppe, z.B. mittels Hydrolyse, z.B. in Gegenwart eines basischen Mittels oder eines sauren Mittels, wie einer Mineralsäure, in eine freie Carboxygruppe überführen.

Ferner kann man in Verbindungen der Formel I, die als Substituenten eine Carboxygruppe aufweisen (insbesondere, sofern $R_3$ von Carboxy verschieden ist), diese, z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z.B. von Zinkchlorid, oder eines wasserbindenden Kondensationsmittels, z.B. eines Carbodiimids, wie von N,N′-Dicyclohexylcarbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen der Formel I, worin die Carboxygruppe in freier Form oder in Salz-, wie Ammonium- oder Metall-, z.B. Alkalimetall-, wie Natrium- oder Kalium-salzform vorliegt, mit einem $C_1$-$C_7$-Alkylhalogenid, z.B. Methyl- oder Ethyl-bromid oder -iodid, oder einem organischen Sulfonsäureester, wie einem entsprechenden $C_1$-$C_7$-Alkylester, z.B. Methansulfonsäure- oder p-Toluolsulfonsäure-methylester oder -ethylester, behandelt.

Verbindungen der Formel I, die als Substituenten eine veresterte Carboxygruppe aufweisen, kann man durch Umesterung, z.B. durch Behandeln mit einem Alkohol, üblicherweise mit einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-$C_1$-$C_7$-alkanoats, -$C_1$-$C_7$-alkanolats oder -cyanids, wie von Natrium-acetat, -methanolat, -ethanolat, -tert.-butanolat oder -cyanid, oder eines geeigneten sauren Mittels, gegebenenfalls unter Entfernung des entstehenden Alkohols, z.B. durch Destillation, in andere Esterverbindungen der Formel I umwandeln. Man kann auch von entsprechenden, sogenannten aktivierten Estern der Formel I ausgehen, die als Substituenten eine aktivierte veresterte Carboxygruppe aufweisen (siehe unten), und diese durch Behandeln mit einem $C_1$-$C_7$-Alkanol in einen anderen Ester umwandeln.

Man kann in Verbindungen der Formel I, die als Substituenten die Carboxygruppe enthalten, diese auch zuerst in ein reaktionsfähiges Derivat, wie ein Anhydrid (auch ein gemischtes Anhydrid), ein Säurehalogenid, z.B. -chlorid (z.B. durch Behandeln mit einem Thionyl-halogenid, z.B. -chlorid), ein Anhydrid mit einem Ameisensäure-ester, z.B. -$C_1$-$C_7$-alkylester (z.B. durch Behandeln eines Salzes, wie eines Ammonium- oder Alkalimetallsalzes, mit einem Halogen-, wie Chlor-ameisensäureester, wie $C_1$-$C_7$-Alkylester), oder einen aktivierten Ester, wie Cyanmethyl-, Nitrophenyl-, z.B. 4-Nitro-phenyl-, oder Polyhalogenphenyl-, z.B. Pentachlorphenyl-ester (z.B. durch Behandeln mit einer entsprechenden Hydroxyverbindung in Gegenwart eines geeigneten Kondensationsmittels, wie von N,N′-Dicyclohexylcarbodiimid) überführen, und ein solches reaktionsfähiges Derivat dann mit einem Amin umsetzen und so zu Amidverbindungen der Formel I gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit einem Amin in Reaktion bringen. Man kann aber auch andere, nicht-aktivierte Ester, wie $C_1$-$C_7$-Alkylester, von Verbindungen der Formel I mit Aminen zur Reaktion bringen.

Weist ein aromatischer Ring als Substituenten ein Wasserstoffatom auf, so kann dieses mit Hilfe eines Halogenierungsmittels in üblicher Weise durch ein Halogenatom ersetzt werden, z.B. mit Brom, Hypobromsäure, einem Acylhypobromit oder einer anderen organischen Bromverbindung, z.B. N-Bromsuccinimid, N-Bromacetamid, N-Bromphthalimid, Pyridiniumperbromid, Dioxandibromid, 1,3-Dibrom-5,5-dimethylhydantoin oder 2,4,4,6-Tetrabrom-2,5-cyclohexandien-1-on, durch Brom oder mit elementarem Chlor, z.B. in einem halogenierten Kohlenwasserstoff, wie Chloroform, und unter Kühlen, z.B. bis auf etwa -10 °C, durch Chlor.

Enthält ein aromatischer Ring eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. einer Mineralsäure, wobei die Reaktionstemperatur vorteilhaft unter etwa 5 °C gehalten wird. Die so erhältliche, in Salzform vorliegende Diazoniumgruppe kann man nach üblichen Verfahren beispielsweise wie folgt substituieren: durch die Hydroxygruppe analog der Phenolverkochung in Gegenwart von Wasser; durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; oder durch Chlor, Brom, [od oder die Cyanogruppe analog der Sandmeyer-Reaktion durch Umsetzung mit entsprechenden Cu([)-Salzen, zunächst unter Kühlen, z.B. auf unter etwa 5 °C, und anschliessendem Erhitzen, z.B. auf etwa 60 ° bis etwa 150 °C.

Enthalten die Verbindungen der Formel I ungesättigte Reste, wie Niederalkenyl oder Niederalkinylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle oder deren Derivate, z.B. Oxide, geeignet sind, wie Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen etwa 1 und etwa 100 at und bei Temperaturen zwischen etwa -80° und etwa +200° C, vor allem zwischen Raumtemperatur und etwa 100° C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Weiterhin kann in Verbindungen der Formel I, worin R Halogen, wie Chlor, bedeutet, Halogen durch Umsetzung mit einem gegebenenfalls substituierten Amin, einem Alkohol oder einem Mercaptan gegen entsprechendes -Z-R′ ausgetauscht werden.

Die Erfindung betrifft insbesondere die in den Beispielen beschriebenen Verfahren.

Salze von Verbindungen der Formel I können in an sich bekannter Weise hergestellt werden. So erhält man beispielsweise Säureadditionssalze von Verbindungen der Formel I durch Behandeln mit einer geeigneten Säure oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden.

Je nach Verfahrensweise bzw. Reaktionsbedingungen können die Verbindungen I mit salzbildenden, insbesondere basischen Eigenschaften, in freier Form oder in Form von Salzen erhalten werden.

Infolge der engen Beziehung zwischen der Verbindung [in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, z. B. zur Kristallisation verwendete, Lösungsmittel einschliessen.

Die Verbindungen I und ihre Salze können, je nach der Wahl der Ausgangsstoffe und Arbeitsweisen, in Form eines der möglichen Isomeren oder als Gemisch derselben, z.B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren oder Racemate aufgetrennt werden, beispielsweise durch fraktionierte Kristallisation. Erhaltene Enantiomerengemische, wie Racemate, lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein-oder Äpfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsgedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen. Neue Ausgangsstoffe und Zwischenprodukte für die Herstellung der Verbindungen I, ihre Verwendung und ein Verfahren zu ihrer Herstellung bilden ebenfalls einen Gegenstand der Erfindung, wobei die Variablen $Z_1$, $Z_2$, $Z_3$, $Z_4$, $R_1$ und $R_2$ die für die Verbindungen I angegebenen Bedeutungen haben. Insbesondere sind Verbindungen der Formel III, in freier Form oder in Salzform, worin $X_2$ Cyano bedeutet, als Ausgangsmaterial bevorzugt.

Die Verbindungen der Formel I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antihypertensiva, verwendet werden.

Die Erfindung betrifft daher gleichfalls pharmazeutische Präparate, die eine Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen oder parenteralen Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1 % bis 100 %, vorzugsweise von etwa 1 % bis etwa 60 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragee-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragee-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung; sie sollen jedoch diese in ihrem Umfang in keiner Weise einschränken. Temperaturen sind in Grad Celsius angegeben.

Die Nomenklatur der den Verbindungen I zugrundeliegenden Azabenzimidazol-Grundgerüste leitet sich

von der entsprechenden Bezeichnung gemäss "Ring Systems Handbook", Ring Systems File I, der American Chemical Society, 1988 Edition, ab; insbesondere wird auf die RF-Nummern 8280, 8284, 8285, 8293, 8334 und 8335 verwiesen.

Beispiel 1: 2-(n-Butyl)-3-(2′-cyanobiphenyl-4-ylmethyl)-3H-imidazo[4,5-b]pyridin (700 mg, 1,91 mmol) und Tributylzinnazid (1,27 g, 3,82 mmol) in o-Xylol (20 ml) werden während 24 Stunden unter Rückfluss gerührt. Das Reaktionsgemisch wird im Vakuum eingedampft und der Rückstand in einem Gemisch von $CH_2Cl_2/CH_3OH/NH_3$ (5/3/1; 30 ml) während 30 Minuten gerührt. Nach erneutem Eindampfen im Vakuum wird der Rückstand mittels Flash-Chromatographie (Kieselgel 60, 40-63 µm, $CH_2Cl_2/CH_3/NH_3$ = 160/10/1) aufgetrennt und das Produkt aus Essigester umkristallisiert. Man erhält so weisse Kristalle von 2-(n-Butyl)-3-[2′-(1H-tetrazol-5-yl)biphenyl-4-ylmethyl]-3H-imidazo[4,5-b]pyridin (Smp.: 139° (Zers.)].

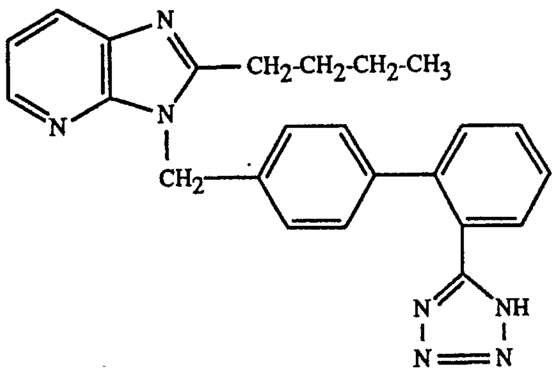

Das Ausgangsmaterial kann z. B. wie folgt hergestellt werden:

a) 2,3-Diaminopyridin (7,7 g, 70,56 mmol) und Valeriansäure (16,6 ml, 141,1 mmol) werden während 20 Stunden bei 160° gerührt. Nach dem Abkühlen wird das Reaktionsgemisch in Essigester gelöst und die Lösung mit gesättigter $NaHCO_3$- und gesättigter NaCl-Lösung gewaschen, getrocknet ($Na_2SO_4$) und im Vakuum eingedampft. Der Rückstand wird in Diethylether suspendiert und abfiltriert. Es verbleiben leicht bräunliche Kristalle von 2-(n-Butyl)-3H-imidazo[4,5-b]pyridin (Smp.: 87-89°).

b) Zu einer Lösung von 2-(n-Butyl)-3H-imidazo[4,5-b]pyridin (1,75 g, 10 mmol) in Dimethylformamid (10 ml) wird NaH (80% in Weissöl, 300 mg, 10 mmol) portionsweise bei Raumtemperatur zugegeben. Nach beendeter Zugabe wird noch 30 Minuten bei Raumtemperatur gerührt und anschliessend eine Lösung von 4-Brommethyl-2′-cyano-biphenyl (2.72g, 10 mmol) in Dimethylformamid (25 ml) zugetropft. Das Reaktionsgemisch wird 12 Stunden bei Raumtemperatur gerührt und anschliessend im Vakuum eingedampft. Der Rückstand wird mit Essigester versetzt und das Gemisch zweimal mit Wasser gewaschen, getrocknet ($Na_2SO_4$) und im Vakuum eingedampft. Flash-Chromatograhie (Kieselgel 60, 40-63 µm, $CH_2Cl_2/CH_3OH$ = 95/5) ergibt 2-(n-Butyl)-3-(2′-cyanobiphenyl-4-ylmethyl)-3H-imidazo[4,5-b]pyridin, welches direkt weiterverarbeitet wird.

Beispiel 2: Ausgehend von 2-(n-Propyl)-3-(2′-cyanobiphenyl-4-ylmethyl)-3H-imidazo[4,5-b]pyridin und Tributylzinnazid erhält man in der in Beispiel 1 beschriebenen Weise 2-(n-Propyl)-3-[2′-(1H-tetrazol-5-yl)-biphenyl-4-ylmethyl]-3H-imidazo[4,5-b]pyridin [weisse Kristalle vom Smp. 161° (Zers.) aus Isopropanol/Essigester].

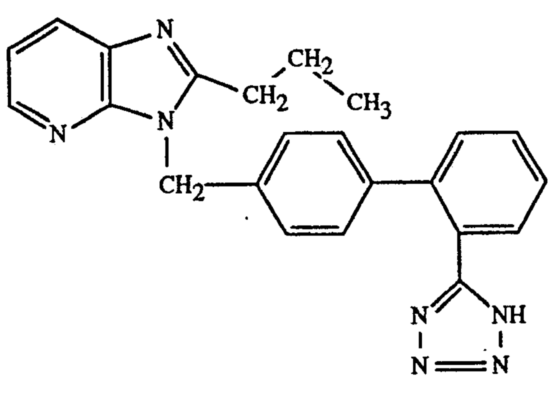

Das Ausgangsmaterial kann beispielsweise wie folgt hergestellt werden:

a) Durch Umsetzung von 2,3-Diaminopyridin mit Buttersäure wird in der in Beispiel 1a) beschriebenen Weise 2-(n-Propyl)-3H-imidazo[4,5-b]pyridin hergestellt. Das Rohprodukt wird aus Essigester/Hexan umkristallisiert (Smp.: 97-99°).

b) Durch Alkylierung von 2-(n-Propyl)-3H-imidazo[4,5-b]pyridin mit 4-Brommethyl-2'- cyano-biphenyl in der in Beispiel 1b) beschriebenen Weise erhält man 2-(n-Propyl)-3-(2'-cyanobiphenyl-4-ylmethyl)-3H-imidazo-[4,5-b]pyridin, welches durch FlashChromatographie (Kieselgel 60, 40-63 µm, Essigester/Hexan = 1/1) gereinigt wird, als Oel. Dieses wird direkt weiterverarbeitet.

Beispiel 3: Ausgehend von 2-(n-Butyl)-3-(2'-cyanobiphenyl-4-ylmethyl)-3H-imidazo[4,5-c]pyridin und Tributylzinnazid erhält man in der in Beispiel 1 beschriebenen Weise 2-(n-Butyl)-3-[2'-(1H-tetrazol-5-yl)-biphenyl-4-ylmethyl]-3H-imidazo[4,5-c]pyridin [weisse Kristalle vom Smp. 120° (Zers.) aus Ethanol/Diethylether].

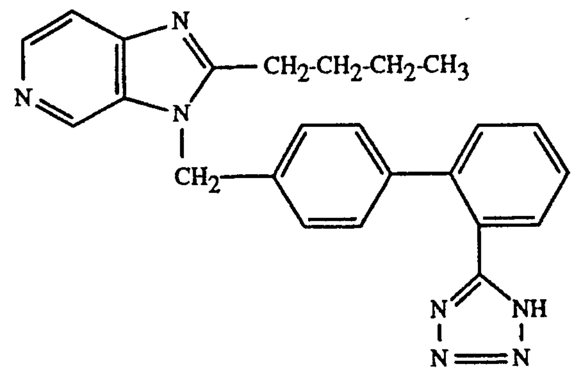

Das Ausgangsmaterial kann beispielsweise wie folgt hergestellt werden:

a) Durch Umsetzung von 3,4-Diaminopyridin mit Valeriansäure wird in der in Beispiel 1a) beschriebenen Weise 2-(n-Butyl)-3H-imidazo[4,5-c]pyridin hergestellt, welches direkt weiterverarbeitet wird.

b) Durch Alkylierung von 2-(n-Butyl)-3H-imidazo[4,5-c]pyridin mit 4-Brommethyl-2'-cyano-biphenyl in der in Beispiel 1b) beschriebenen Weise entsteht ein Gemisch von 2-(n-Butyl)-3-(2'-cyanobiphenyl-4-ylmethy)-3H-imidazo[4,5-c]pyridin und 2-(n-Butyl)-1-(2'-cyanobiphenyl-4-ylmethyl)-1H-imidazo[4,5-c]pyridin, welches mittels Flash-Chromatographie (Kieselgel 60, 40-63 µm, $CH_2Cl_2/CH_3OH$ = 98/2) in die einzelnen Komponenten aufgetrennt wird. Die gewünschte Komponente wird direkt weiterverarbeitet.

Beispiel 4: Ausgehend von 2-(n-Butyl)-1-(2'-cyanobiphenyl-4-ylmethyl)-1H-imidazo[4,5-c]pyridin [Beispiel 3b)] und Tributylzinnazid erhält man in der in Beispiel 1 beschriebenen Weise 2-(n-Butyl)-1-[2'-(1H-tetrazol-5-yl)biphenyl-4-ylmethyl]-1H- imidazo[4,5-c]pyridin [weisse Kristalle vom Smp. 179° (Zers.) aus Ethanol/Essigester].

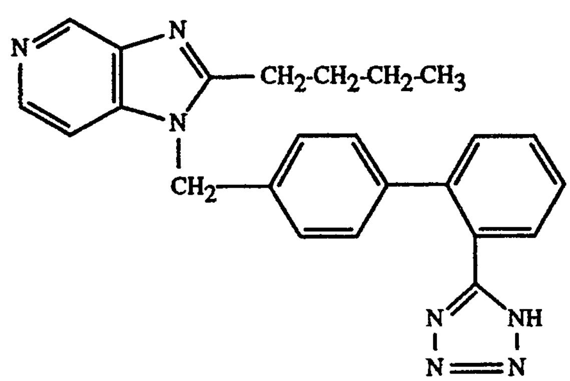

Beispiel 5: Ausgehend von 2-(n-Heptafluorpropyl)-3-(2'-cyanobiphenyl-4-ylmethyl)-3H-imidazo[4,5-b]-pyridin und Tributylzinnazid erhält man in der in Beispiel 1 beschriebenen Weise 2-(n-Heptafluorpropyl)-3-[2'-(1H-tetrazol-5-yl)biphenyl-4-ylmethyl]-3H-imidazo[4,5-b]pyridin [Smp.: 120-121° (aus Essigester/Cyclohexan)].

Das Ausgangsmaterial kann beispielsweise wie folgt hergestellt werden:

a) Durch Umsetzung von 2,3-Diaminopyridin mit Perfluorbuttersäure wird in der in Beispiel 1a) beschriebenen Weise 2-(n-Heptafluorpropyl)-3H-imidazo[4,5-b]pyridin hergestellt [Smp.: 203-204° (aus Essigester/Hexan)].

b) Durch Alkylierung von 2-(n-Heptafluorpropyl)-3H-imidazo[4,5-b]pyridin mit 4-Brommethyl-2′-cyano-biphenyl in der in Beispiel 1b) beschriebenen Weise und Flash-Chromatographie (Kieselgel 60, 40-63 µm, Hexan/Essigester = 4/1) erhält man 2-(n-Heptafluorpropyl)-3-(2,-cyanobiphenyl-4-ylmethyl)-3H-imidazo[4,5-b]pyridin, welches direkt weiterverarbeitet wird.

Beispiel 6: Ausgehend von 8-(n-Butyl)-9-(2′-cyanobiphenyl-4-ylmethyl)-9H-purin und Tributylzinnazid erhält man in der in Beispiel 1 beschriebenen Weise 8-(n-Butyl)-9-[2′-(1H-tetrazol-5-yl)biphenyl-4-ylmethyl]-9H-purin [Smp.: 155° (Zers.); aus Essigester].

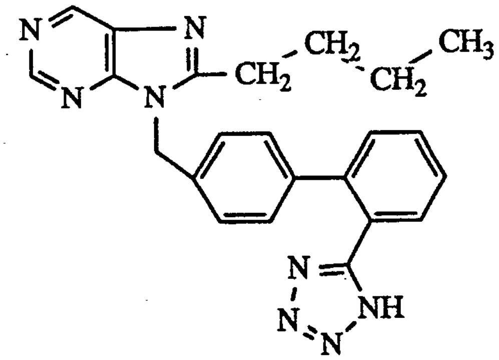

Das Ausgangsmaterial kann beispielsweise wie folgt hergestellt werden:

a) Durch Umsetzung von 4,5-Diaminopyrimidin mit Valeriansäure wird in der in Beispiel 1a) beschriebenen Weise 8-(n-Butyl)-9H-purin hergestellt, welches direkt weiterverarbeitet wird.

b) Durch Alkylierung von 8-(n-Butyl)-9H-purin mit 4-Brommethyl-2′-cyano-biphenyl in der in Beispiel 1b) beschriebenen Weise und Flash-Chromatographie (Kieselgel 60, 40-63 µm, $CH_2Cl_2/CH_3OH$ = 95/5) erhält man 8-(n-Butyl)-9-(2′-cyanobiphenyl-4-ylmethyl)-9H-purin, welches direkt weiterverarbeitet wird.

Beispiel 7: In analoger Weise wie in einem der vorstehenden Beispiele beschrieben kann man herstellen:

2-(n-Butyl)-1-[2′-(1H-tetrazol-5-yl)biphenyl-4-ylmethyl]- 1H-imidazo(4,5-b]pyrazin,
6-(n-Butyl)-7-[2′-(1H-tetrazol-5-yl)biphenyl-4-ylmethy]-7H-imidazo[4,5-c]pyridazin,
2-(n-Butyl)-1-(2′-(1H-tetrazol-5-yl)biphenyl-4-ylmethy]-1H-imidazo[4,5-d]pyridazin,
6-(n-Butyl)-5-[2′-(1H-tetrazol-5-yl)biphenyl-4-ylmethyl]-5H-imidazo[4,5-c]pyridazin,
8-(n-Butyl)-7-[2′-(1H-tetrazol-5-yl)biphenyl-4-ylmethyl]-7H-purin,
2-(n-Butyl)-1-[2′-(1H-tetrazol-5-yl)biphenyl-4-ylmethyl]-1H-imidazo[4,5-b]pyridin,
2-[(E)-But-1-en-1-yl]-3-[2′-(1H-tetrazol-5-yl)biphenyl-4-ylmethyl]-3H-imidazo[4,5-b]pyridin,
2-[(E)-Propen-1-yl]-3-[2′-(1H-tetrazol-5-yl)biphenyl-4ylmethyl]-3H-imidazo[4,5-b]pyridin,
2-(n-Butyl)-3-(2′-carboxybiphenyl-4-ylmethyl]-3H-imidazo(4,5-b]pyridin,
2-(n-propyl)3-(2′-carboxybiphenyl-4-ylmethyl]-3H-imidazo(4,5-b]pyridin,
2-(n-Heptafluoropropyl)-3-[2′-carboxybiphenyl-4-ylmethyl]-3H-imidazo[4,5b]pyridin,
2-(n-Butyl)-3-[2′-carboxybiphenyl-4-ylmethyl]-3H-imidazo[4,5-c]pyridin,
2-(n-Butyl)-1-[2′-carboxybiphenyl-4-ylmethyl]-1H-imidazo[4,5-c]pyridin,
8-(n-Butyl)-9-[2′-carboxybiphenyl-4-ylmethyl]-9H-purin,
2-(n-Butyl)-1-[2′-carboxybiphenyl-4-ylmethyl]-1H-imidazo[4,5-b]pyrazin,
6-(n-Butyl)7-[2′-carboxybiphenyl-4-ylmethyl]-7H-imidazo[4,5-c]pyridazin,
2-(n-Butyl)-1-[2′-carboxybiphenyl-4-ylmethyl] -1H-imidazo[4,5-d]pyridazin,
6-(n-Butyl)-5-[2′-carboxybiphenyl-4-ylmethyl]-5H-imidazo(4,5-c]pyridazin,
8(n-Butyl)-7-[2′-carboxybiphenyl-4-ylmethyl]-7H-purin,
2-(n-Butyl)-1-[2′-carboxybiphenyl-4-ylmethyl]-1H-imidazo[4,5-b]pyridin,
2-[(E)-But-1-en-1-yl]-3-[2′-carboxybiphenyl-4-ylmethyl]-3H-imidazo[4,5-b]pyridin, und
2-[(E)-Propen-1-yl]-3-(2′-carboxybiphenyl-4-ylmethyl]-3H-imidazo[4,5-b]pyridin.

Beispiel 8: Tabletten, enthaltend je SO mg Wirkstoff, z.B. 2-(n-Butyl)-3-[2′-(1H-tetrazol-5-yl)biphenyl-4-ylmethyl]-3H-imidazo[4,5-b]pyridin, können wie folgt hergestellt werden:

| Zusammensetzung (für 10000 Tabletten): | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 9: Lacktabletten, enthaltend je 100 mg Wirkstoff, z.B. 2-(n-Butyl)-3-[2'-(1H-tetrazol-5-yl)-biphenyl-4-ylmethyl]-3H-imidazo(4,5-b]pyridin, können wie folgt hergestellt werden:

| Zusammensetzung (für 1000 Tabletten): | |
|---|---|
| Wirkstoff | 100,00 g |
| Lactose | 100,00 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Dichlormethan lackiert (Endgewicht der Lacktablette: 283 mg).

Beispiel 10: In analoger Weise wie in den Beispielen 8 und 9 beschrieben können auch Tabletten und Lacktabletten, enthaltend eine andere Verbindung der Formel I oder ein pharmazeutisch verwendbares Salz einer Verbindung der Formel I, z.B. gemäss einem der Beispiele 1 bis 7, hergestellt werden.

## Ansprüche

1. Eine Verbindung der Formel

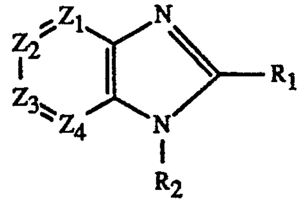

(I),

worin eine oder zwei der Variablen $Z_1$, $Z_2$, $Z_3$ und $Z_4$ für N und die anderen für C(R) stehen, wobei R Halogen, Acyl, gegebenenfalls verestertes oder amidiertes Carboxy oder 5-Tetrazolyl bedeutet oder R für -Z-R' steht, worin Z für eine Bindung oder für O, $S(O)_m$ oder NH steht, R' Wasserstoff oder einen aliphatischen Kohlenwasserstoffrest, der gegebenenfalls durch Halogen, Hydroxy, gegebenenfalls substituiertes Amino oder gegebenenfalls verestertes oder amidiertes Carboxy substituiert ist und der gegebenenfalls durch O oder $S(O)_m$ unterbrochen ist, bedeutet und der Index m jeweils für 0, 1 oder 2 steht, $R_1$ einen gegebenenfalls durch Halogen oder Hydroxy substituierten aliphatischen Kohlenwasserstoffrest oder einen cycloaliphatischen oder araliphatischen Kohlenwasserstoffrest bedeutet und $R_2$ für die Gruppe der Formel

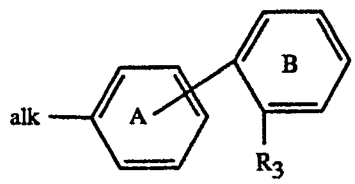

(Ia)

steht, in der alk einen zweiwertigen aliphatischen Kohlenwasserstoffrest bedeutet, $R_3$ COOH, $SO_3H$, Halogenalkansulfonylamino, $PO_2H_2$; $PO_3H_2$ oder 5-Tetrazolyl bedeutet und entweder die Ringe A und B unabhängig voneinander gegebenenfalls durch Halogen, einen gegebenenfalls durch Hydroxy oder Halogen substituierten, gegebenenfalls durch O unterbrochenen, aliphatischen Kohlenwasserstoffrest, gegebenenfalls durch einen aliphatischen Alkohol verethertes Hydroxy oder gegebenenfalls verestertes oder amidiertes Carboxy substituiert sind oder der Ring A durch 5-Tetrazolyl substituiert ist und der Ring B gegebenenfalls wie unmittelbar vorstehend angegeben substituiert ist, in freier Form oder in Salzform.

2. Eine Verbindung gemäss Anspruch 1 der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, oder 5-Tetrazolyl bedeutet oder R für -Z-R' steht, worin Z für eine Bindung oder für O, $S(O)_m$ oder NH steht, R' Wasserstoff oder jeweils gegebenenfalls durch Halogen, durch Hydroxy, durch Amino, welches gegebenenfalls wie unmittelbar vorstehend in der Definition der Aminogruppe des Carbamoylrestes R angegeben substituiert ist, durch Carboxy, welches gegebenenfalls wie unmittelbar vorstehend angegeben verestert ist, oder durch Carbamoyl, in welchem die Aminogruppe gegebenenfalls wie unmittelbar vorstehend angegeben substituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkylthioniederalkyl, Niederalkylthioniederalkenyl, Niederalkylthioniederalkinyl, Niederalkansulfinylniederalkyl, Niederalkansulfonylniederalkyl, Niederalkenylthioniederalkyl, Niederalkenylsulfinylniederalkyl, Niederalkenylsulfonylniederalkyl, Niederalkinylthioniederalkyl, Niederalkinylsulfinylniederalkyl oder Niederalkinylsulfonylniederalkyl bedeutet und der Index m für 0, 1 oder 2 steht, $R_1$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet und $R_2$ für die Gruppe der Formel Ia steht, in der alk Niederalkylen oder Niederalkenylen bedeutet, $R_3$ COOH, $SO_3H$, Halogenniederalkansulfonylamino, $PO_2H_2$, $PO_3H_2$ oder 5-Tetrazolyl bedeutet und entweder die Ringe A und B unabhängig voneinander gegebenenfalls durch Halogen, durch jeweils gegebenenfalls durch Halogen oder Hydroxy substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl oder Niederalkenyloxyniederalkinyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxy niederalkinyl ableitet, oder durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, substituiert sind oder der Ring A durch 5-Tetrazolyl substituiert ist und der Ring B gegebenenfalls wie unmittelbar vorstehend angegeben substituiert ist in freier Form oder in Salzform.

3. Eine Verbindung gemäss Anspruch 1 der Formel I, worin eine oder zwei der Variablen $Z_1$, $Z_2$, $Z_3$ und $Z_4$

für N und die anderen für C(R) stehen, wobei R Halogen, Niederalkanoyl, Carboxy, Niederalkoxycarbonyl, Niederalkoxyniederalkoxycarbonyl, Carbamoyl, in welchem die Aminogruppe gegebenenfalls durch Niederalkyl oder Phenylniederalkyl unabhängig voneinander mono- oder disubstituiert oder durch Niederalkylen disubstituiert ist, oder R für -Z-R′ steht, worin Z für eine Bindung oder für O steht und R′ Wasserstoff oder jeweils gegebenenfalls durch Halogen, durch Hydroxy, durch Amino, welches gegebenenfalls durch Niederalkyl oder Phenylniederalkyl unabhaengig voneinander mono- oder disubstituiert oder durch Niederalkylen disubstituiert ist, durch Carboxy, durch Niederalkoxycarbonyl oder durch Niederalkoxyniederalkoxycarbonyl substituiertes Niederalkyl oder Niederalkoxyniederalkyl bedeutet, $R_1$ jeweils gegebenenfalls durch Halogen oder Hydroxy substituiertes Niederalkyl oder Niederalkenyl, 3- bis 7-gliedriges Cycloalkyl oder Phenylniederalkyl bedeutet und $R_2$ für die Gruppe der Formel Ia steht, in der alk Niederalkylen bedeutet, $R_3$ COOH oder 5-Tetrazolyl bedeutet und die Ringe A und B unabhängig voneinander gegebenenfalls durch Halogen, durch gegebenenfalls durch Halogen oder Hydroxy substituiertes Niederalkyl, durch Niederalkoxy, durch Carboxy oder durch Niederalkoxycarbonyl substituiert sind, in freier Form oder in Salzform.

4. Eine Verbindung gemäss Anspruch 1 der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, Niederalkoxycarbonyl, gegebenenfalls durch Niederalkyl mono- oder disubstituiertes Carbamoyl oder 5-Tetrazolyl bedeutet oder R für -Z-R′ steht, worin Z für eine Bindung oder für O, $S(O)_m$ oder NH steht, m für 0, 1 oder 2 steht und R′ Wasserstoff oder gegebenenfalls durch Halogen, Hydroxy oder Amino substituiertes Niederalkyl bedeutet, $R_1$ Niederalkyl, Niederalkenyl, Hydroxyniederalkyl, Halogenniederalkyl, 3- bis 7-gliedriges Cycloalkyl oder Phenylniederalkyl bedeutet und $R_2$ für die Gruppe der Formel Ia steht, in der alk Niederalkylen bedeutet, $R_3$ COOH oder 5-Tetrazolyl bedeutet und die Ringe A und B unabhängig voneinander gegebenenfalls durch Halogen, durch gegebenenfalls durch Halogen oder Hydroxy substituiertes Niederalkyl, durch Niederalkoxy, durch Carboxy oder durch Niederalkoxycarbonyl substituiert sind, in freier Form oder in Salzform.

5. Eine Verbindung gemäss einem der Ansprüche 1 bis 4 der Formel I, worin $R_2$ für die Gruppe der Formel

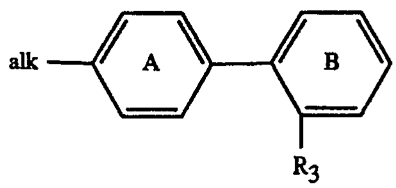

(Ib)

steht, in freier Form oder in Salzform.

6. Eine Verbindung gemäss Anspruch 1 der Formel I, worin eine oder zwei der Variablen $Z_1$, $Z_2$, $Z_3$ und $Z_4$ für N und die anderen für C(R) stehen, wobei R Wasserstoff, Halogen, Carboxy, Niederalkoxycarbonyl, Niederalkyl, Halogenniederalkyl, Hydroxyniederalkyl oder Niederalkoxy bedeutet, $R_1$ Niederalkyl, Niederalkenyl, Hydroxyniederalkyl, Halogenniederalkyl, 3- bis 7-gliedriges Cycloalkyl oder Phenylniederalkyl bedeutet und $R_2$ für die Gruppe der Formel Ib steht, in der alk Niederalkylen bedeutet, $R_3$ COOH oder 5-Tetrazolyl bedeutet und entweder die Ringe A und B unabhängig voneinander gegebenenfalls durch Halogen, Niederalkyl, Halogenniederalkyl, Niederalkoxy, Carboxy oder Niederalkoxycarbonyl substituiert sind oder der Ring A durch 5-Tetrazolyl substituiert ist und der Ring B gegebenenfalls wie unmittelbar vorstehend angegeben substituiert ist, in freier Form oder in Salzform.

7. Eine Verbindung gemäss einem der Ansprüche 1 bis 6 der Formel I, worin $R_2$ fuer die Gruppe der Formel Ia oder Ib steht und alk Methylen bedeutet, in freier Form oder in Salzform.

8. Eine Verbindung gemäss Anspruch 1 der Formel I, worin eine oder zwei der Variablen $Z_1$, $Z_2$, $Z_3$ und $Z_4$ für N und die anderen für CH stehen, insbesondere stehen $Z_1$, $Z_2$ und $Z_3$ für CH und $Z_4$ für N, $R_1$ Niederalkyl, insbesondere mit bis und mit 4 C-Atomen, Niederalkenyl, insbesondere mit 3 bis und mit 5 C-Atomen, oder Halogenniederalkyl, insbesondere mit bis und mit 4 C-Atomen und Halogen mit einer Atomnummer bis und mit 35, bedeutet und $R_2$ für die Gruppe der Formel Ib steht, in der alk Methylen bedeutet, $R_3$ COOH oder 5-Tetrazolyl ist und die Ringe A und B unabhängig voneinander unsubstituiert oder in zweiter Linie durch Halogen, insbesondere mit einer Atomnummer bis und mit 35, Niederalkyl, insbesondere mit bis und mit 4 C-Atomen, oder Niederalkoxy, insbesondere mit bis und mit 4 C-Atomen, substituiert sind, in freier Form oder in Salzform.

9. Eine Verbindung gemäss einem der Ansprüche 1 bis 8 der Formel I, worin eine oder zwei der Variablen $Z_1$, $Z_2$, $Z_3$ und $Z_4$ für N und die anderen für CH stehen oder worin insbesondere eine der Variablen $Z_2$, $Z_3$ und $Z_4$ fuer N und $Z_1$ und die anderen der Variablen $Z_2$, $Z_3$ und $Z_4$ fuer C(R), insbesondere CH, stehen oder worin $Z_1$ und $Z_3$ fuer C(R), insbesondere CH, und $Z_2$ und $Z_4$ fuer N stehen, in freier Form oder in

Salzform.

10. Eine Verbindung gemäss Anspruch 1 der Formel I, worin $Z_1$, $Z_2$ und $Z_3$ für CH und $Z_4$ für N oder $Z_1$ und $Z_3$ fuer CH und $Z_2$ und $Z_4$ fuer N stehen, $R_1$ Niederalkyl, insbesondere mit bis und mit 4 C-Atomen, Niederalkenyl, insbesondere mit 3 bis und mit 5 C-Atomen, oder Halogenniederalkyl, insbesondere mit bis und mit 4 C-Atomen und Halogen mit einer Atomnummer bis und mit 35, edeutet und $R_2$ für die Gruppe der Formel Ib steht, in der alk Methylen bedeutet, $R_3$ COOH oder 5-Tetrazolyl ist und die Ringe A und B unabhängig voneinander unsubstituiert oder in zweiter Linie durch Halogen, insbesondere mit einer Atomnummer bis und mit 35, Niederalkyl, insbesondere mit bis und mit 4 C-Atomen, oder Niederalkoxy, insbesondere mit bis und mit 4 C-Atomen, substituiert sind, in freier Form oder in Salzform.

11. Eine Verbindung gemäss Anspruch 1 der Formel I, worin $Z_1$, $Z_2$ und $Z_3$ für CH stehen, $Z_4$ für N steht, $R_1$ $C_3$-$C_4$-Alkyl, bedeutet und $R_2$ für die Gruppe der Formel Ib steht, in der alk Methylen bedeutet, $R_3$ 5-Tetrazolyl bedeutet und die Ringe A und B unsubstituiert sind, in freier Form oder in Salzform.

12. 2-(n-Butyl)-3-[2′-(1H-tetrazol-5-yl)biphenyl-4-ylmethyl]-3H-imidazo[4,5-b]pyridin, in freier Form oder in Salzform.

13. 2-(n-Propyl)-3-[2′-(1H-tetrazol-5-yl)biphenyl-4-ylmethyl]-3H-imidazo[4,5-b]pyridin, in freier Form oder in Salzform.

14. 2-(n-Butyl)-3-[2′-(1H-tetrazol-5-yl)biphenyl-4-ylmethyl]-3H-imidazo[4,5-c]pyridin, in freier Form oder in Salzform.

15. 2-(n-Butyl)-1-[2′-(1H-tetrazol-5-yl)biphenyl-4-ylmethyl]-1H-imidazo[4,5-c]pyridin, in freier Form oder in Salzform.

16. 2-(n-Heptafluorpropyl)-3-[2′-(1H-tetrazol-5-yl)biphenyl-4-ylmethyl]-3H-imidazo[4,5-b]pyridin, in freier Form oder in Salzform.

17. 8-(n-Butyl)-9-[2′-(1H-tetrazol-5-yl)biphenyl-4-ylmethyl]-9H-purin, in freier Form oder in Salzform.

18. 2-(n-Butyl)-1-[2′-(1H-tetrazol-5-yl)biphenyl-4-ylmethyl]-1H-imidazo[4,5-b]pyridin,
6-(n-Butyl)-7-[2′-(1H-tetrazol-5-yl)biphenyl-4-ylmethyl]-7H-imidazo[4,5-c]pyridazin,
2-(n-Butyl)-1-[2′-(1H-tetrazol-5-yl)biphenyl-4-ylmethyl]-1H-imidazo[4,5-d]pyridazin,
6-(n-Butyl)-5-[2′-(1H-tetrazol-5-yl)biphenyl-4-ylmethyl]-5H-imidazo[4,5-c]pyridazin,
8-(n-Butyl)-7-[2′-(1H-tetrazol-5-yl)biphenyl-4-ylmethyl]-7H-purin,
2-(n-Butyl)-1-[2′-(1H-tetrazol-5-yl)biphenyl-4-ylmethyl]-1H-imidazo[4,5-b]pyridin,
2-[(E)-But-1-en-1-yl]-3-[2′-(1H-tetrazol-5-yl)biphenyl-4-ylmethyl]-3H-imidazo[4,5-b]pyridin,
2-[(E)-Propen-1-yl]-3-[2′-(1H-tetrazol-5-yl)biphenyl-4-ylmethyl]-3H-imidazo[4,5-b]pyridin,
2-(n-Butyl)-3-[2′-carboxybiphenyl-4-ylmethyl]-3H-imidazo[4,5-b]pyridin,
2-(n-Propyl)-3-[2′-carboxybiphenyl-4-ylmethyl]-3H-imidazo[4,5-b]pyridin,
2-(n-Heptafluoropropyl)-3-[2′-carboxybiphenyl-4-ylmethyl]-3H-imidazo[4,5-b]pyridin,
2-(n-Butyl)-3-[2′-carboxybiphenyl-4-ylmethyl]-3H-imidazo[4,5-c]pyridin,
2-(n-Butyl)-1-[2′-carboxybiphenyl-4-ylmethyl]-1H-imidazo[4,5-c]pyridin,
8-(n-Butyl)-9-[2′-carboxybiphenyl-4-ylmethyl]-9H-purin,
2-(n-Butyl)-1-[2′-carboxybiphenyl-4-ylmethyl]-1H-imidazo[4,5-b]pyrazin,
6-(n-Butyl)-7-[2′-carboxybiphenyl-4-ylmethyl]-7H-imidazo[4,5-c]pyridazin,
2-(n-Butyl)-1-[2′-carboxybiphenyl-4-ylmethyl]-1H-imidazo[4,5-d]pyridazin,
6-(n-Butyl)-5-carboxybiphenyl-4-ylmethyl]-5H-imidazo[4,5-c]pyridazin,
8-(n Butyl)-7-[2′-carboxybiphenyl-4-ylmethyl]-7H-purin,
2-(n-Butyl)-1-[2′-carboxybiphenyl-4-ylmethyl]-1H-imidazo[4,5-b)pyridin,
2 [(E)-But-1-en-1-yl]-3-[2′-carboxybiphenyl-4-ylmethyl]-3H-imidazo[4,5 b]pyridin, oder
2 [(E)-Propen-1-yl]-3-[2′-carboxybiphenyl-4-ylmethyl]-3H-imidazo[4,5 b]pyridin, jeweils in freier Form oder in Salzform.

19. Eine Verbindung gemäss einem der Ansprüche 1 bis 18, in freier Form oder in Form eines pharmazeutisch verwendbaren Salzes, zur Anwendung in einem Verfahren zur therapeutischen Behandlung des menschlichen oder tierischen Körpers.

20. Eine Verbindung gemäss einem der Ansprüche 4 bis 6 und 12 bis 18, in freier Form oder in Form eines pharmazeutisch verwendbaren Salzes, zur Anwendung in einem Verfahren zur therapeutischen Behandlung des menschlichen oder tierischen Körpers.

21. Eine Verbindung gemäss einem der Ansprüche 1 bis 20, in freier Form oder in Form eines pharmazeutisch verwendbaren Salzes, zur Anwendung als Antihypertensivum.

22. Eine Verbindung gemäss einem der Ansprüche 4 bis 6, 12 bis 18 und 20, in freier Form oder in Form eines pharmazeutisch verwendbaren Salzes, zur Anwendung als Antihypertensivum.

23. Ein pharmazeutisches Präparat, als Wirkstoff enthaltend eine Verbindung gemäss einem der Ansprüche 1 bis 22, in freier Form oder in Form eines pharmazeutisch verwendbaren Salzes, gegebenenfalls neben

üblichen pharmazeutischen Hilfsstoffen.

24. Ein pharinazeutisches Präparat, als Wirkstoff enthaltend eine Verbindung gemäss einem der Ansprüche 4 bis 6, 12 bis 18, 20 und 22, in freier Form oder in Form eines pharmazeutisch verwendbaren Salzes, gegebenenfalls neben üblichen pharmazeutischen Hilfsstoffen.

25. Ein antihypertensiv wirksames pharmazeutisches Präparat gemäss Anspruch 23 oder 24, dadurch gekennzeichnet, dass man einen antihypertensiv wirksamen Wirkstoff wählt.

26. Ein antihypertensiv wirksames pharmazeutisches Präparat gemäss Anspruch 24, dadurch gekennzeichnet, dass man einen antihypertensiv wirksamen Wirkstoff wählt.

27. Verfahren zur Herstellung einer Verbindung gemäss einem der Ansprüche 1 bis 18 der Formel I, in freier Form oder in Salzform, dadurch gekennzeichnet, dass man

a) eine Verbindung der Formel

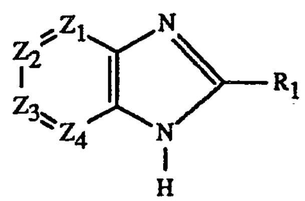

(IIa)

oder ein Salz davon mit einer Verbindung der Formel

$X_1$-$R_2$ (IIb)

oder einem Salz davon, worin $X_1$ reaktionsfähiges verestertes Hydroxy bedeutet, umsetzt oder

b) in einer Verbindung der Formel

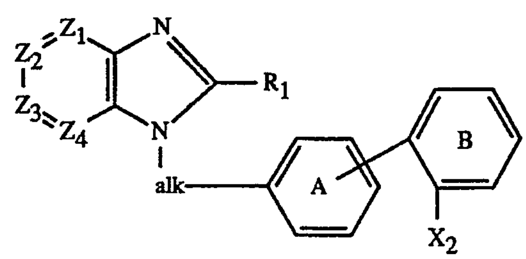

(III)

oder einem Salz davon, worin $X_2$ einen in die Variable $R_3$ überführbaren Rest bedeutet, $X_2$ in die Variable $R_3$ überführt oder

c) eine Verbindung der Formel

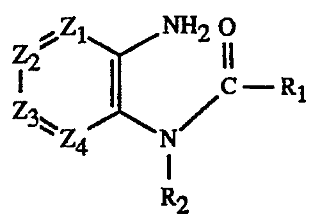

(IV)

oder ein Salz davon cyclisiert und jeweils, wenn erwünscht, eine verfahrensgemäss oder auf andere Weise erhältliche Verbindung der Formel I in freier Form oder in Salzform in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

28. Verfahren zur Herstellung eines pharmazeutischen Präparats gemäss einem der Ansprüche 23 bis 26, dadurch gekennzeichnet, dass man den Wirkstoff, gegebenenfalls unter Beimischung von üblichen pharmazeutischen Hilfsstoffen, zu einem pharmazeutischen Präparat verarbeitet.

29. Verfahren gemäss Anspruch 28 zur Herstellung eines antihypertensiv wirksamen pharmazeutischen Präparats gemäss Anspruch 25 oder 26, dadurch gekennzeichnet, dass man einen antihypertensiv wirksamen Wirkstoff wählt.

30. Verwendung einer Verbindung gemäss einem der Ansprüche 1 bis 22, in freier Form oder in Form eines pharmazeutisch verwendbaren Salzes, zur Herstellung eines pharmazeutischen Präparats.

31. Verwendung einer Verbindung gemäss einem der Ansprüche 1 bis 22, in freier Form oder in Form eines pharmazeutisch verwendbaren Salzes, zur Herstellung eines pharmazeutischen Präparats auf nicht-chemischem Wege.

32. Verwendung einer Verbindung gemäss Anspruch 30 oder 31 zur Herstellung eines Antihypertensivums.

Patentansprüche für folgende Vertragsstaaten: ES, GR:

1. Verfahren zur Herstellung einer Verbindung der Formel

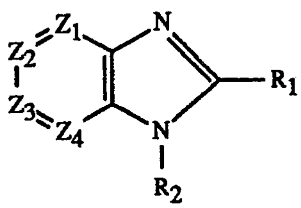

(I),

worin eine oder zwei der Variablen $Z_1$, $Z_2$, $Z_3$ und $Z_4$ für N und die anderen für C(R) stehen, wobei R Halogen, Acyl, gegebenenfalls verestertes oder amidiertes Carboxy oder 5-Tetrazolyl bedeutet oder R für -Z-R′ steht, worin Z für eine Bindung oder für O, $S(O)_m$ oder NH steht, R′ Wasserstoff oder einen aliphatischen Kohlenwasserstoffrest, der gegebenenfalls durch Halogen, Hydroxy, gegebenenfalls substituiertes Amino oder gegebenenfalls verestertes oder amidiertes Carboxy substituiert ist und der gegebenenfalls durch O oder $S(O)_m$ unterbrochen ist, bedeutet und der Index m jeweils für 0, 1 oder 2 steht, $R_1$ einen gegebenenfalls durch Halogen oder Hydroxy substituierten aliphatischen Kohlenwasserstoffrest oder einen cycloaliphatischen oder araliphatischen Kohlenwasserstoffrest bedeutet und $R_2$ für die Gruppe der Formel

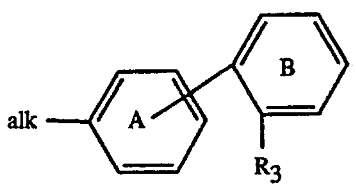

(Ia)

steht, in der alk einen zweiwertigen aliphatischen Kohlenwasserstoffrest bedeutet, $R_3$ COOH, $SO_3H$, Halogenalkansulfonylamino, $PO_2H_2$, $PO_3H_2$ oder 5-Tetrazolyl bedeutet und entweder die Ringe A und B unabhängig voneinander gegebenenfalls durch Halogen, einen gegebenenfalls durch Hydroxy oder Halogen substituierten, gegebenenfalls durch O unterbrochenen, aliphatischen Kohlenwasserstoffrest, gegebenenfalls durch einen aliphatischen Alkohol verethertes Hydroxy oder gegebenenfalls verestertes oder amidiertes Carboxy substituiert sind oder der Ring A durch 5-Tetrazolyl substituiert ist und der Ring B gegebenenfalls wie unmittelbar vorstehend angegeben substituiert ist, in freier Form oder in Salzform, dadurch gekennzeichnet, dass man

a) eine Verbindung der Formel

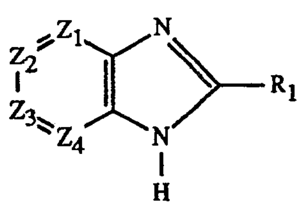

(IIa)

oder ein Salz davon mit einer Verbindung der Formel

$X_1$-$R_2$ (IIb)

oder einem Salz davon, worin $X_1$ reaktionsfähiges verestertes Hydroxy bedeutet, umsetzt oder

b) in einer Verbindung der Formel

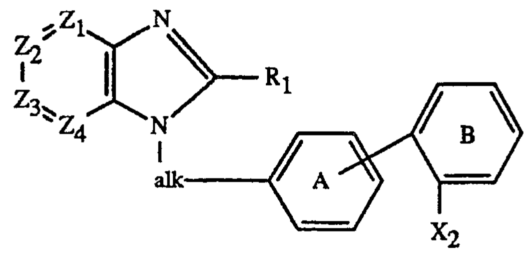

(III)

oder einem Salz davon, worin $X_2$ einen in die Variable $R_3$ überführbaren Rest bedeutet, $X_2$ in die Variable $R_3$ überführt oder

c) eine Verbindung der Formel

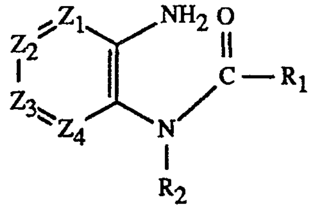

(IV)

oder ein Salz davon cyolisiert und jeweils, wenn erwünscht, eine verfahrensgemäss oder auf andere Weise erhältliche Verbindung der Formel I in freier Form oder in Salzform in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

2. Verfahren gemäss Anspruch 1, dadurch gekennzeichnet, dass man eine Verbindung der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, oder 5-Tetrazolyl bedeutet oder R für -Z-R′ steht, worin Z für eine Bindung oder für O, $S(O)_m$ oder NH steht, R′ Wasserstoff oder jeweils gegebenenfalls durch Halogen, durch Hydroxy, durch Amino, welches gegebenenfalls wie unmittelbar vorstehend in der Definition der Aminogruppe des Carbamoylrestes R angegeben substituiert ist, durch Carboxy, welches gegebenenfalls wie unmittelbar vorstehend angegeben verestert ist, oder durch Carbamoyl, in welchem die Aminogruppe gegebenenfalls wie unmittelbar vorstehend angegeben substituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkylthioniederalkyl, Niederalkylthioniederalkenyl, Niederalkylthioniederalkinyl, Niederalkansulfinylniederalkyl, Niederalkansulfonylniederalkyl, Niederalkenylthioniederalkyl, Niederalkenylsulfinylniederalkyl, Niederalkenylsulfnylniederalkyl, Niederalkinylthioniederalkyl, Niederalkinylsulfinylniederalkyl oder Niederalkinylsulfonylniederalkyl bedeutet und der Index m für 0, 1 oder 2 steht, $R_1$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet und $R_2$ für die Gruppe der Formel Ia steht, in der alk Niederalkylen oder Niederalkenylen bedeutet, $R_3$ COOH, $SO_3H$, Halogenniederalkansulfonylamino, $PO_2H_2$, $PO_3H_2$ oder 5-Tetrazolyl bedeutet und entweder die Ringe A und B unabhängig voneinander gegebenenfalls durch Halogen, durch jeweils gegebenenfalls durch Halogen oder Hydroxy substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl oder Niederalkenyloxyniederalkinyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, substituiert sind oder der Ring A durch 5-Tetrazolyl substituiert ist und der Ring B gegebenenfalls wie unmittelbar vorstehend angegeben substituiert ist, in freier Form oder in Salzform, herstellt.

3. Verfahren gemäss Anspruch 1, dadurch gekennzeichnet, dass man eine Verbindung der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, Niederalkoxycarbonyl, Niederalkoxyniederalkoxycarbonyl, Carbamoyl, in welchem die Aminogruppe gegebenenfalls durch Niederalkyl oder Phenylniederalkyl unabhängig voneinander mono- oder disubstituiert oder durch Niederalkylen disubstituiert ist, oder R für -Z-R′ steht, worin Z für eine Bindung oder für O steht und R′ Wasserstoff oder jeweils gegebenenfalls durch Halogen, durch Hydroxy, durch Amino, welches gegebenenfalls durch Niederalkyl oder Phenylniederalkyl unabhaengig voneinander mono- oder disubstituiert oder durch Niederalkylen disubstituiert ist, durch Carboxy, durch Niederalkoxycarbonyl oder durch Niederalkoxyniederalkoxycarbonyl substituiertes Niederalkyl oder Niederalkoxyniederalkyl bedeutet, $R_1$ jeweils gegebenenfalls durch Halogen oder Hydroxy substituiertes Niederalkyl oder Niederalkenyl, 3- bis 7-gliedriges Cycloalkyl oder Phenylniederalkyl bedeutet und $R_2$ für die Gruppe der Formel Ia steht, in der alk Niederalkylen bedeutet, $R_3$ COOH oder 5-Tetrazolyl bedeutet und die Ringe A und B unabhängig voneinander gegebenenfalls durch Halogen, durch gegebenenfalls durch Halogen oder Hydroxy substituiertes Niederalkyl, durch Niederalkoxy, durch Carboxy oder durch Niederalkoxycarbonyl substituiert sind, in freier Form oder in Salzform, herstellt.

4. Verfahren gemäss Anspruch 1, dadurch gekennzeichnet, dass man eine Verbindung der Formel I, worin eine oder zwei der Variablen $Z_1$, $Z_2$, $Z_3$ und $Z_4$ für N und die anderen für C(R) stehen, wobei R Halogen, Niederalkanoyl, Carboxy, Niederalkoxycarbonyl, gegebenenfalls durch Niederalkyl mono- oder disubstituiertes Carbamoyl oder 5-Tetrazolyl bedeutet oder R für -Z-R′ steht, worin Z für eine Bindung oder für O, $S(O)_m$ oder NH steht, m für 0, 1 oder 2 steht und R′ Wasserstoff oder gegebenenfalls durch Halogen, Hydroxy oder Amino substituiertes Niederalkyl bedeutet, $R_1$ Niederalkyl, Niederalkenyl, Hydroxyniederalkyl, Halogenniederalkyl, 3- bis 7-gliedriges Cycloalkyl oder Phenylniederalkyl bedeutet und $R_2$ für die Gruppe der Formel Ia steht, in der alk Niederalkylen bedeutet, $R_3$ COOH oder 5-Tetrazolyl bedeutet und die Ringe A und B unabhängig voneinander gegebenenfalls durch Halogen, durch gegebenenfalls durch Halogen oder Hydroxy substituiertes Niederalkyl, durch Niederalkoxy, durch Carboxy oder durch Niederalkoxycarbonyl substituiert sind, in freier Form oder in Salzform, herstellt.

5. Verfahren gemäss einem der Ansprüche 1 bis 4, dadurch gekennzeichnet, dass man eine Verbindung der Formel I, worin $R_2$ für die Gruppe der Formel

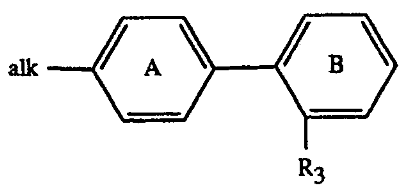

(Ib)

steht, in freier Form oder in Salzform, herstellt.

6. Verfahren gemäss Anspruch 1, dadurch gekennzeichnet, dass man eine Verbindung der Formel I, worin eine oder zwei der Variablen $Z_1$, $Z_2$, $Z_3$ und $Z_4$ für N und die anderen für C(R) stehen, wobei R Wasserstoff, Halogen, Carboxy, Niederalkoxycarbonyl, Niederalkyl, Halogenniederalkyl, Hydroxyniederalkyl oder Niederalkoxy bedeutet, $R_1$ Niederalkyl, Niederalkenyl, Hydroxyniederalkyl, Halogenniederalkyl, 3- bis 7-gliedriges Cycloalkyl oder Phenylniederalkyl bedeutet und $R_2$ für die Gruppe der Formel Ib steht, in der alk Niederalkylen bedeutet, $R_3$ COOH oder 5-Tetrazolyl bedeutet und entweder die Ringe A und B unabhängig voneinander gegebenenfalls durch Halogen, Niederalkyl, Halogenniederalkyl, Niederalkoxy, Carboxy oder Niederalkoxycarbonyl substituiert sind oder der Ring A durch 5-Tetrazolyl substituiert ist und der Ring B gegebenenfalls wie unmittelbar vorstehend angegeben substituiert ist, in freier Form oder in Salzform, herstellt.

7. Verfahren gemäss einem der Ansprüche 1 bis 6, dadurch gekennzeichnet, dass man eine Verbindung der Formel I, worin $R_2$ fuer die Gruppe der Formel Ia oder Ib steht und alk Methylen bedeutet, in freier Form oder in Salzform, herstellt.

8. Verfahren gemäss Anspruch 1, dadurch gekennzeichnet, dass man eine Verbindung der Formel I, worin

eine oder zwei der Variablen $Z_1$, $Z_2$, $Z_3$ und $Z_4$ für N und die anderen für CH stehen, insbesondere stehen $Z_1$, $Z_2$ und $Z_3$ für CH und $Z_4$ für N, $R_1$ Niederalkyl, insbesondere mit bis und mit 4 C-Atomen, Niederalkenyl, insbesondere mit 3 bis und mit 5 C-Atomen, oder Halogenniederalkyl, insbesondere mit bis und mit 4 C-Atomen und Halogen mit einer Atomnummer bis und mit 35, bedeutet und $R_2$ für die Gruppe der Formel Ib steht, in der alk Methylen bedeutet, $R_3$ COOH oder 5-Tetrazolyl ist und die Ringe A und B unabhängig voneinander unsubstituiert oder in zweiter Linie durch Halogen, insbesondere mit einer Atomnummer bis und mit 35, Niederalkyl, insbesondere mit bis und mit 4 C-Atomen, oder Niederalkoxy, insbesondere mit bis und mit 4 C-Atomen, substituiert sind, in freier Form oder in Salzform, herstellt.

9. Verfahren gemäss einem der Ansprüche 1 bis 8, dadurch gekennzeichnet, dass man eine Verbindung der Formel I, worin eine oder zwei der Variablen $Z_1$, $Z_2$, $Z_3$ und $Z_4$ für N und die anderen für CH stehen oder worin insbesondere eine der Variablen $Z_2$, $Z_3$ und $Z_4$ fuer N und $Z_1$ und die anderen der Variablen $Z_2$, $Z_3$ und $Z_4$ fuer C(R), insbesondere CH, stehen oder worin $Z_1$ und $Z_3$ fuer C(R), insbesondere CH, und $Z_3$ und $Z_4$ fuer N stehen, in freier Form oder in Salzform, herstellt.

10. Verfahren gemäss Anspruch 1, dadurch gekennzeichnet, dass man eine Verbindung der Formel I, worin $Z_1$, $Z_2$ und $Z_3$ für CH und $Z_4$ für N oder $Z_1$ und $Z_2$ fuer CH und $Z_2$ und $Z_4$ fuer N stehen, $R_1$ Niederalkyl, insbesondere mit bis und mit 4 C-Atomen, Niederalkenyl, insbesondere mit 3 bis und mit 5 C-Atomen, oder Halogenniederalkyl, insbesondere mit bis und mit 4 C-Atomen und Halogen mit einer Atomnummer bis und mit 35, edeutet und $R_2$ für die Gruppe der Formel Ib steht, in der alk Methylen bedeutet, $R_3$ COOH oder 5-Tetrazolyl ist und die Ringe A und B unabhängig voneinander unsubstituiert oder in zweiter Linie durch Halogen, insbesondere mit einer Atomnummer bis und mit 35, Niederalkyl, insbesondere mit bis und mit 4 C-Atomen, oder Niederalkoxy, insbesondere mit bis und mit 4 C-Atomen, substituiert sind, in freier Form oder in Salzform, herstellt.

11. Verfahren gemäss Anspruch 1, dadurch gekennzeichnet, dass man eine Verbindung der Formel I, worin $Z_1$, $Z_2$ und $Z_3$ für CH stehen, $Z_4$ für N steht, $R_1$ $C_3$-$C_4$-Alkyl, bedeutet und $R_2$ für die Gruppe der Formel Ib steht, in der alk Methylen bedeutet, $R_3$ 5-Tetrazolyl bedeutet und die Ringe A und B unsubstituiert sind, in freier Form oder in Salzform, herstellt.

12. Verfahren gemäss Anspruch 1, dadurch gekennzeichnet, dass man 2-(n-Butyl)-3-[2′-(1H-tetrazol-5-yl)-biphenyl-4-ylmethyl]-3H-imidazo[4,5-b]pyridin, in freier Form oder in Salzform, herstellt.

13. Verfahren gemäss Anspruch 1, dadurch gekennzeichnet, dass man 2-(n-Propyl)-3-[2′-(1H-tetrazol-5-yl)-biphenyl-4-ylmethyl]-3H-imidazo[4,5-b]pyridin, in freier Form oder in Salzform, herstellt.

14. Verfahren gemäss Anspruch 1, dadurch gekennzeichnet, dass man 2-(n-Butyl)-3-[2′-(1H-tetrazol-5-yl)-biphenyl-4-ylmethyl]-3H-imidazo[4,5-c]pyridin, in freier Form oder in Salzform, herstellt.

15. Verfahren gemäss Anspruch 1, dadurch gekennzeichnet, dass man 2-(n-Butyl)-1-[2′-(1H-tetrazol-5-yl)-biphenyl-4-ylmethyl]-1H-imidazo[4,5-c]pyridin, in freier Form oder in Salzform, herstellt.

16. Verfahren gemäss Anspruch 1, dadurch gekennzeichnet, dass man 2-(n-Heptafluorpropyl)-3-[2′-(1H-tetrazol-5-yl)biphenyl-4-ylmethyl]-3H-imidazo[4,5-b]pyridin, in freier Form oder in Salzform, herstellt.

17. Verfahren gemäss Anspruch 1, dadurch gekennzeichnet, dass man 8-(n-Butyl)-9-[2′-(1H-tetrazol-5-yl)-biphenyl-4-ylmethyl]-9H-pyrazin, in freier Form oder in Salzform, herstellt.

18. Verfahren gemäss Anspruch 1, dadurch gekennzeichnet, dass man
2-(n-Butyl)-1-[2′-(1H-tetrazol-5-yl)biphenyl-4-ylmethyl]-1H-i midazo[4,5-b]pyrazin,
6-(n-Butyl)-7-[2′-(1H-tetrazol-5-yl)biphenyl-4-ylmethyl]-7H-imidazo[4,5-c]pyridazin,
2-(n-Butyl)-1-[2′-(1H-tetrazol-5-yl)biphenyl-4-ylmethyl]-1H-imidazo[4,5-d]pyridazin,
6-(n-Butyl)-5-[2′-(1H-tetrazol-5-yl)biphenyl-4-ylmethyl]-5H-imidazo[4,5-c]pyridazin,
8-(n-Butyl)-7-[2′-(1H-tetrazol-5-yl)biphenyl-4-ylmethyl]-7H-purin,
2-(n-Butyl)-1-[2′-(1H-tetrazol-5-yl)biphenyl-4-ylmethyl]-1H-imidazo[4,5-b]pyridin,
2-[(E)-But-1-en-1-yl]-3-[2′-(1H-tetrazol-5-yl)biphenyl-4-ylmethyl]-3H-imidazo[4,5b]pyridin,
2-[(E)-Propen-1-yl]-3-[2′-(1H-tetrazol-5-yl)biphenyl-4-ylmethyl]-3H-imidazo[4,5-b]pyridin,
2(n-Butyl)-3-[2′-carboxybiphenyl-4-ylmethyl]-3H-imidazo[4,5-b]pyridin,
2-(n-Propyl)3-[2′-carboxybiphenyl-4-ylmethyl]-3H-imidazo[4,5-b]pyridin,
2(n-Heptafluoropropyl)-3-[2′-carboxybiphenyl-4-ylmethyl]-3H-imidazo[4,5-b]pyridin,
2-(n-Butyl)-3-[2′-carboxybiphenyl-4-ylmethyl]-3H-imidazo[4,5-c]pyridin,
2-(n-Butyl)-1-[2′-carboxybiphenyl-4-ylmethyl]-1H-imidazo[4,5-c]pyridin,
8-(n-Butyl)-9-[2′-carboxybiphenyl-4-ylmethyl]-9H-purin,
2-(n-Butyl)-1-[2′-carboxybiphenyl-4-ylmethyl]-1H-imidazo[4,5-b]pyrazin,
6(n-Butyl)-7-[2′-carboxybiphenyl-4-ylmethyl]-7H-imidazo[4,5c]pyridazin,
2-(n-Butyl)-1-[2′-carboxybiphenyl-4-ylmethyl]-1H-imidazo[4,5-d]pyridazin,
6-(n-Butyl)-5-[2′-carboxybiphenyl-4-ylmethyl] -5H-imidazo[4,5-c]pyridazin,
8-(n-Butyl)-7-[2′-carboxybiphenyl-4-ylmethyl]-7H-purin,

2-(n-Butyl)-1-[2′-carboxybiphenyl-4-ylmethyl]-1H-imidazo[4,5-b]pyridin,
2-[(E)But-1-en-1-yl]-3-[2′-carboxybiphenyl-4-ylmethyl]-3H-imidazo[4,5-b]pyridin, oder
2-[(E)-Propen-1-yl]-3-[2′-carboxybiphenyl-4-ylmethyl]-3H-imidazo[4,5-b]pyridin, jeweils in freier Form oder in Salzform, herstellt.

19. Verfahren zur Herstellung eines pharmazeutischen Präparats, dadurch gekennzeichnet, dass man
a) eine Verbindung der Formel

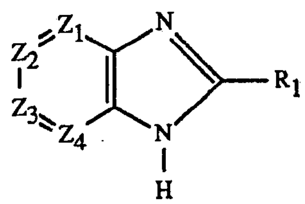

(IIa)

oder ein Salz davon mit einer Verbindung der Formel
$X_1$-$R_2$ (IIb)
oder einem Salz davon, worin $X_1$ reaktionsfähiges verestertes Hydroxy bedeutet, umsetzt oder
b) in einer Verbindung der Formel

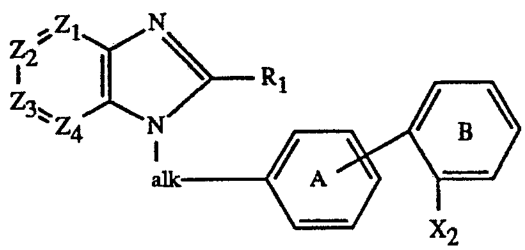

(III)

oder einem Salz davon, worin $X_2$ einen in die Variable $R_3$ überführbaren Rest bedeutet, $X_2$ in die Variable $R_3$ überführt oder
c) eine Verbindung der Formel

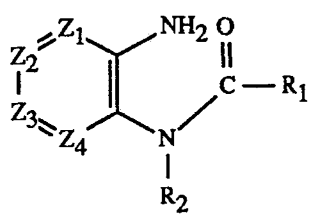

(IV)

oder ein Salz davon cyclisiert und jeweils, wenn erwünscht, eine verfahrensgemäss oder auf andere Weise erhältliche Verbindung der Formel I in freier Form oder in pharmazeutisch verwendbarer Salzform in eine andere Verbindung der Formel I überführt, ein verfahrensgemäss erhältliches Gemisch von Isomeren auftrennt und das gewünschte Isomere isoliert und/oder eine verfahrensgemäss erhältliche freie Verbindung der Formel I in ein pharmazeutisch verwendbares Salz oder ein verfahrensgemäss erhältliches pharmazeutisch verwendbares Salz einer Verbindung der Formel I in die freie Verbindung der Formel I oder in ein anderes pharmazeutisch verwendbares Salz überführt und eine auf diese Weise erhaltene Verbindung der Formel

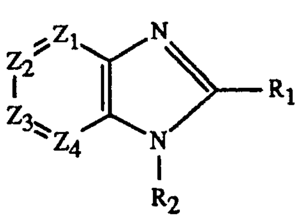

(I),

worin eine oder zwei der Variablen $Z_1$, $Z_2$, $Z_3$ und $Z_4$ für N und die anderen für C(R) stehen, wobei R Halogen, Acyl, gegebenenfalls verestertes oder amidiertes Carboxy oder 5-Tetrazolyl bedeutet oder R für -Z-R' steht, worin Z für eine Bindung oder für O, $S(O)_m$ oder NH steht, R' Wasserstoff oder einen aliphatischen Kohlenwasserstoffrest, der gegebenenfalls durch Halogen, Hydroxy, gegebenenfalls substituiertes Amino oder gegebenenfalls verestertes oder amidiertes Carboxy substituiert ist und der gegebenenfalls durch O oder $S(O)_m$ unterbrochen ist, bedeutet und der Index m jeweils für 0, 1 oder 2 steht, $R_1$ einen gegebenenfalls durch Halogen oder Hydroxy substituierten aliphatischen Kohlenwasserstoffrest oder einen cycloaliphatischen oder araliphatischen Kohlenwasserstoffrest bedeutet und $R_2$ für die Gruppe der Formel

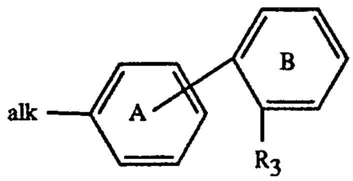

(Ia)

steht, in der alk einen zweiwertigen aliphatischen Kohlenwasserstoffrest bedeutet, $R_3$ COOH, $SO_3H$, Halogenalkansulfonylamino, $PO_2H_2$, $PO_3H_2$ oder 5-Tetrazolyl bedeutet und entweder die Ringe A und B unabhängig voneinander gegebenenfalls durch Halogen, einen gegebenenfalls durch Hydroxy oder Halogen substituierten, gegebenenfalls durch O unterbrochenen, aliphatischen Kohlenwasserstoffrest, gegebenenfalls durch einen aliphatischen Alkohol verethertes Hydroxy oder gegebenenfalls verestertes oder amidiertes Carboxy substituiert sind oder der Ring A durch 5-Tetrazolyl substituiert ist und der Ring B gegebenenfalls wie unmittelbar vorstehend angegeben substituiert ist, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

20. Verfahren zur Herstellung eines pharmazeutischen Präparats, dadurch gekennzeichnet, dass man eine Verbindung, erhältlich gemäss einem der Ansprüche 1 bis 18, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

21. Verfahren zur Herstellung eines pharmazeutischen Präparats gemäss Anspruch 20, dadurch gekennzeichnet, dass man eine Verbindung, erhältlich gemäss einem der Ansprüche 4 bis 6 und 12 bis 18, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

22. Verfahren gemäss einem der Ansprüche 19 bis 21 zur Herstellung eines antihypertensiv wirksamen pharmazeutischen Präparats, dadurch gekennzeichnet, dass man einen antihypertensiv wirksamen Wirkstoff wählt.

23. Verfahren gemäss Anspruch 21 zur Herstellung eines antihypertensiv wirksamen pharmazeutischen Präparats, dadurch gekennzeichnet, dass man einen antihypertensiv wirksamen Wirkstoff wählt.

24. Verwendung einer Verbindung, erhältlich gemäss einem der Ansprüche 1 bis 18, in freier Form oder in Form eines pharmazeutisch verwendbaren Salzes, zur Herstellung eines pharmazeutischen Präparats.

25. Verwendung einer Verbindung, erhältlich gemäss einem der Ansprüche 1 bis 18, in freier Form oder in Form eines pharmazeutisch verwendbaren Salzes, zur Herstellung eines pharmazeutischen Präparats auf nicht-chemischem Wege.

26. Verwendung einer Verbindung gemäss Anspruch 24 oder 25 zur Herstellung eines Antihypertensivums.